# EUROPEAN PATENT SPECIFICATION

(11) **EP 3 474 870 B1**
(45) Date of publication and mention of the grant of the patent: **27.07.2022**
(21) Application number: 17735417.2
(22) Date of filing: 23.06.2017
(51) Int. Cl.: A61K 35/74, A61K 45/00, A61P 19/02

(54) **MEANS AND METHODS TO TREAT INFLAMMATION-ASSOCIATED DISORDERS OR CONDITIONS**
MITTEL UND VERFAHREN ZUR BEHANDLUNG VON ENTZÜNDUNGSBEDINGTEN ERKRANKUNGEN ODER LEIDEN
MOYENS ET PROCÉDÉS DE TRAITEMENT DE TROUBLES OU ÉTATS ASSOCIÉS À L'INFLAMMATION

(30) Priority: 23.06.2016 EP 16176039
(43) Date of publication of application: 01.05.2019
(73) Proprietor: VIB VZW, 9052 Gent (BE); Universiteit Gent, 9000 Gent (BE); Katholieke Universiteit Leuven K.U. Leuven R&D, 3000 Leuven (BE)
(72) Inventor: RAES, Jeroen, 2600 Berchem (BE); ELEWAUT, Dirk, 9070 Heusden (BE); TITO-TADEO, Raul Yhossef, 3020 Herent (BE); HUYS, Geert, 8700 Aarsele (BE); VEREECKE, Lars, 9971 Lembeke (BE)
(86) International application number: PCT/EP2017/065592
(87) International publication number: WO 2017/220802

(56) References cited:
- WO-A1-2013/037068
- WO-A1-2017/109059
- John D Reveille: "Spondyloarthritis (Spondyloarthropathies)", , 1 January 2010 (2010-01-01), XP055399482, Retrieved from the Internet: URL:https://www.rchsd.org/documents/2014/0 2/spondyloarthritis.pdf [retrieved on 2017-08-17]
- C. CASÉN ET AL: "Deviations in human gut microbiota: a novel diagnostic test for determining dysbiosis in patients with IBS or IBD", ALIMENTARY PHARMACOLOGY & THERAPEUTICS., vol. 42, no. 1, 14 May 2015 (2015-05-14), pages 71-83, XP055351398, GB ISSN: 0269-2813, DOI: 10.1111/apt.13236
- ORNA NITZAN: "Role of antibiotics for treatment of inflammatory bowel disease", WORLD JOURNAL OF GASTROENTEROLOGY, vol. 22, no. 3, 1 January 2016 (2016-01-01), page 1078, XP055399568, CN ISSN: 1007-9327, DOI: 10.3748/wjg.v22.i3.1078
- F. MORIO ET AL: "Antimicrobial Susceptibilities and Clinical Sources of Dialister Species", ANTIMICROBIAL AGENTS AND CHEMOTHERAPY, vol. 51, no. 12, 8 October 2007 (2007-10-08), pages 4498-4501, XP055399577, ISSN: 0066-4804, DOI: 10.1128/AAC.00538-07
- MARY-ELLEN COSTELLO ET AL: "Brief Report: Intestinal Dysbiosis in Ankylosing Spondylitis : Gut Microbiome and AS-Related Genes", ARTHRITIS & RHEUMATOLOGY (HOBOKEN), vol. 67, no. 3, 25 February 2015 (2015-02-25), pages 686-691, XP055399344, US ISSN: 2326-5191, DOI: 10.1002/art.38967

## Description

### FIELD OF THE INVENTION

The present invention relates to the field of inflammation-associated disorders or conditions, more particularly to gut inflammation and/or joint inflammation, even more particularly to spondyloarthritis. Provided herein are means and methods to treat, prevent and/or reduce the severity of inflammation-associated disorders or conditions in a subject in need thereof.

### BACKGROUND

Spondyloarthritis (SpA), also known as spondyloarthropathy, is a chronic inflammatory condition involving the axial skeleton and/or peripheral joints. SpA is characterized by axial inflammation (sacroiliitis and spondylitis) and/or asymmetrical peripheral arthritis/enthesitis. Patients with SpA often present extra-articular manifestations such as psoriasis, uveitis and inflammatory bowel disease (IBD). A reciprocal overlap exists between IBD and SpA: about 5-10% of SpA patients develop IBD. Conversely, up to 30% of IBD patients may develop SpA-like articular inflammation. Importantly, up to 50% of all SpA patients show intestinal inflammation on a microscopic level, without associated gastrointestinal symptoms. Two types of inflammation can be distinguished based on histomorphological characteristics: an acute type resembling infectious enterocolitis, and a chronic type, quite similar to early Crohn's disease (CD). Previous studies have demonstrated a clinical relationship between gut and joint inflammation in SpA, with remission of joint inflammation being associated with disappearance of gut inflammation and vice versa (Abraham & Medzhitov, 2011, Gastroenterology, 140:1729-1737). Assessment of disease severity in SpA patients was initially based on using single-item measures (e.g. pain, stiffness, erythrocyte sedimentation rate [ESR], C-reactive protein [CRP], patient/physician global assessment) and composite indices (e.g. Bath Ankylosing Spondylitis Disease Activity Index - BASDAI). However, both have limitations because they measure only part of disease activity, and are fully patient or physician oriented. The composite index Ankylosing Spondylitis Disease Activity Score (ASDAS) is a new instrument to measure disease activity in early spondyloarthritis and to establish thresholds as targets of treatment in patients with spondyloarthritis. It reflects disease activity from both the patient and the physician perspective, which are known to be inherently different. It includes the following items: back pain, duration of morning stiffness, patient global assessment of disease activity, peripheral pain or swelling, and an acute phase reactant, preferably CRP, alternatively ESR.

The treatment goals for SpA are maintenance of physical function, control of disease activity and prevention of radiographic progression. Today, treatments consist primarily anti-inflammatory and long-lasting therapies which may not impact the underlying causes of the disease. Furthermore, effective preventive treatments for spondyloarthritis are virtually non-existent. There is thus a high unmet need for improved means and methods for the treatment and/or prevention of spondyloarthritis.

In contrast to SpA where the true cause of disease is not unraveled yet, the current hypothesis of IBD pathogenesis involves an inappropriate immune response to dysbalanced intestinal bacteria in a genetically susceptible host (Lees et al. 2011, Gut, 60:1739-1753). The reciprocity between bowel and joint inflammation and the overlap with IBD, suggests that intestinal microbiota might play a role in SpA pathogenesis as well. Dysbiosis with a decrease in intestinal microbial diversity has been consistently found in IBD patients (for example, Huttenhower et al. 2014, Immunity, 40: 843-854) and an altered intestinal microbiota composition has also been described in unaffected relatives of CD patients (Joossens et al. 2011, Gut, 60:631-637). Although completely unrelated to SpA, also for rheumatoid arthritis a link with the gut microbiome was found, more precisely *Dialister invisus* was found to be a beneficial bacterium for rheumatoid arthritis (CN 104 546 939 A). CN 104 546 939 A further disclosed that treatment of mice with *Dialister invisus* reduced their macroscopic scores for arthritis. A gut microbial profile distinct from unaffected controls was described for SpA patients (Costello et al. 2014, Arthritis & Rheumatology, Nov 21) and the use of the antibiotic ciprofloxacin has been described against a specific form of spondyloarthritis, namely reactive arthritis induced by Chlamydia trachomatis (Reveille, 2010, Patient fact sheet provided by the American College of Rheumatology, www.rchsd.org/documents/2014/02/spondyloarthritis.pdf), yet the relation between gut microbial composition, gut histology and disease activity markers in SpA remains unknown.

### SUMMARY OF THE INVENTION

In the current application, it was found that gut inflammation in SpA patients is strongly associated with intestinal microbiota composition. Moreover, we found a positive correlation between the abundance of *Dialister* (a genus of Firmicutes bacteria classified within the class Negativicutes) and disease activity as reflected by ASDAS and BASDAI. Furthermore, a SpA mice model treated with stool samples obtained from SpA patients having high abundance of *Dialister* spp. showed significantly higher disease scores compared to mice treated with stool samples with low abundance of *Dialister.* Also, mice treated with stool samples with a low abundance of *Dialister* showed significantly lower disease scores compared to the control mice. Based on these novel findings, *Dialister* can thus not only be used as a marker of inflammation but also treatment options targeting *Dialister* spp. are disclosed in this application. Modulating *Dialister* abundance in the gut can positively affect the inflammation status of a subject, and can lead to decreased gut and/or joint inflammation in patients, in particular SpA patients. Additionally, at a sub-genus level it was surprisingly found that the high abundant *Dialister* OTUs (operational taxonomic units) in SpA patients differ from those in healthy subjects. In other words, within the genus *Dialister* there are disease associated and non-disease associated OTUs. We also demonstrated that the population of native enteric *Dialister* OTUs in SpA patients comprises disease associated OTUs.

The present invention provides a composition comprising at least one Dialister spp. for use in treating, preventing or reducing the severity of spondyloarthritis (SpA) or of gut and/or joint inflammation in SpA patients, wherein the at least one Dialister spp. is selected from the list consisting of OTU4552 comprising SEQ ID No. 11, OTU6938 comprising SEQ ID No. 12, OTU1378 comprising SEQ ID No. 13, OTU605 comprising SEQ ID No. 14 and OTU223 comprising SEQ ID No. 15. In one embodiment, said at least one Dialister spp. originates from bacterial collections. In a particular embodiment, said bacterial collections are obtained from fecal samples and/or microbiota obtained from intestinal biopsies or other clinical biopsies. In another embodiment, said fecal samples and/or biopsies are obtained from a healthy subject.

In another embodiment, said composition is a fecal transplant or an oral formulation. In yet another embodiment, the composition further comprises an anti-inflammatory and/or an analgesic drug. In another embodiment, SpA is selected from ankylosing spondylitis, non-radiographic axial SpA, reactive arthritis, psoriatic arthritis, enteropathic arthritis, undifferentiated spondyloarthritis, juvenile idiopathic arthritis or juvenile-onset SpA.

The invention also provides a screening method to identify a compound that reduces the intestinal abundance of disease associated Dialister spp. selected from OTU60 comprising SEQ ID No. 57 or OTU62 comprising SEQ ID No. 56, said method comprising the following steps:
- contacting isolated disease associated Dialister spp. from subjects suffering from or at risk of SpA or of gut and/or joint inflammation with a compound in an in vitro environment;
- selecting a compound that inhibits growth of at least one of said disease associated Dialister spp. In one embodiment, said screening method is provided wherein SpA is selected from ankylosing spondylitis, non-radiographic axial SpA, reactive arthritis, psoriatic arthritis, enteropathic arthritis, undifferentiated spondyloarthritis, juvenile idiopathic arthritis or juvenile-onset SpA.

### BRIEF DESCRIPTION OF THE DRAWINGS

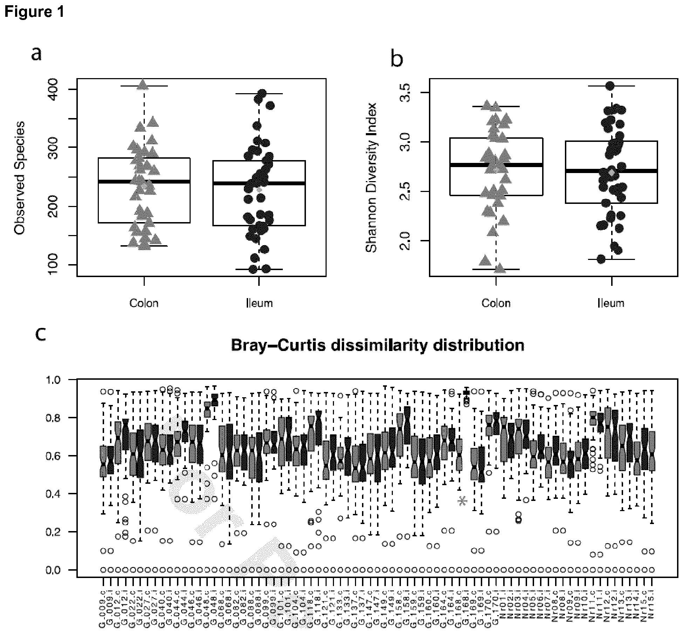

**Figure 1****. Alpha and beta diversity of the biopsies.** Microbial diversity comprises richness (observed species) and evenness (how relative abundance or biomass is distributed among species). While richness is a direct measurement of OTUs (a), the Shannon Diversity Index estimates evenness (b). Our analysis did not detect significant differences between richness and evenness of colonic and ileal biopsies. Orange diamonds are the mean in plots (a) and (b). Bray Curtis dissimilarity distances (BCD) of the colonic and ileal biopsies showed the variation in the community composition (beta diversity). In (c), we show the BCD of all pair wise comparison between a given biopsy (ileum or colon) and the rest of the biopsies. Empty circles represent outlier on the box plots, for all the samples there are outliers at 0 (Bray-Curtis distance against the same sample). After performing Welch t-test with 1000 permutations to test if the mean of the BCD of ileum and colonic biopsies of the same individual is significant different, only one comparison results significant (sample G.168 in orange ^{∗}).

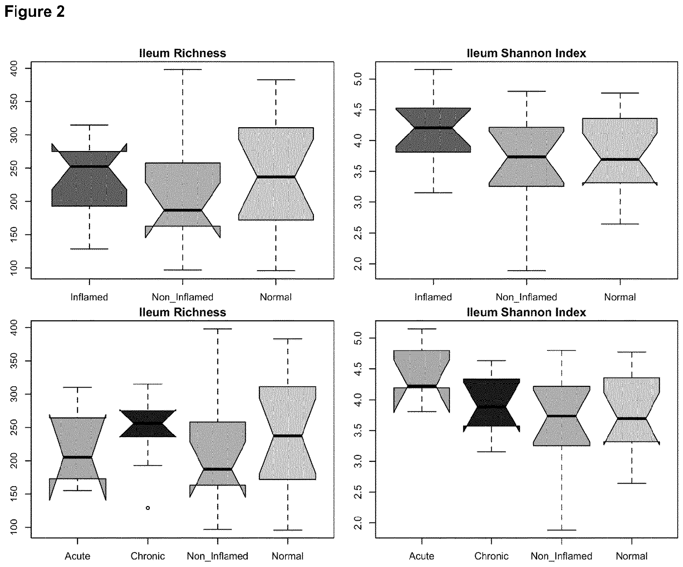

**Figure 2****. Microbial diversity and inflammation in** ileal **biopsies from SpA patients (Inflamed (Acute/Chronic)** - **Non_Inflamed) and healthy controls (Normal).** Microbial richness grouped within inflammation subtypes, revealed higher richness in chronic than in acute and non-inflamed biopsies. The Shannon Diversity Index showed a correlation between the type of inflammation and diversity: higher in acute, followed by chronic inflammation and lower levels of diversity in non-inflamed and healthy.

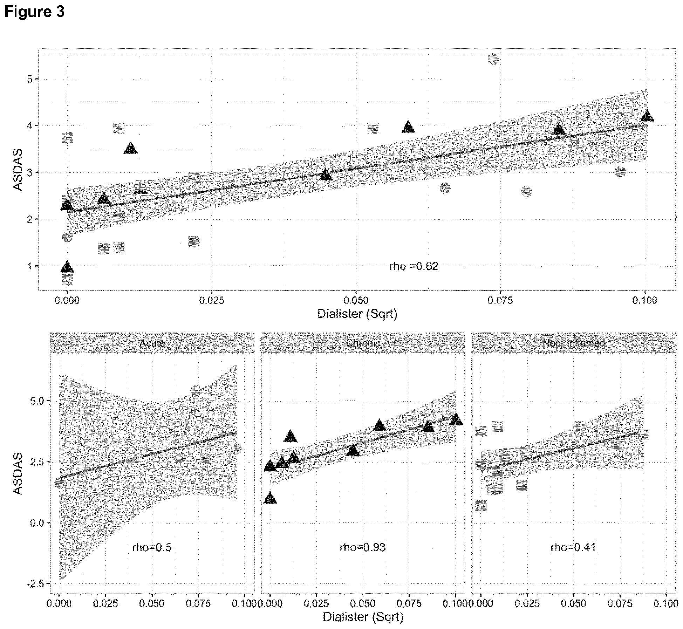

**Figure** 3. **Spearman's rank correlation of the abundance of the genus** *Dialister* **with** disease **activity.** Upper row: Dialister positively correlated with ASDAS values (rho = 0.62, p=0.0003, q=0.0096). Lower row: Results divided by bowel histology: acute (rho = 0.50, p=0.225, q=0.225), chronic (rho = 0.93, p=0.0001, q=0.0004) and non-inflamed (rho = 0.41, p=0.0842, q=0.1264). *Dialister* abundance is presented by square root (Sqrt) to facilitate visualization

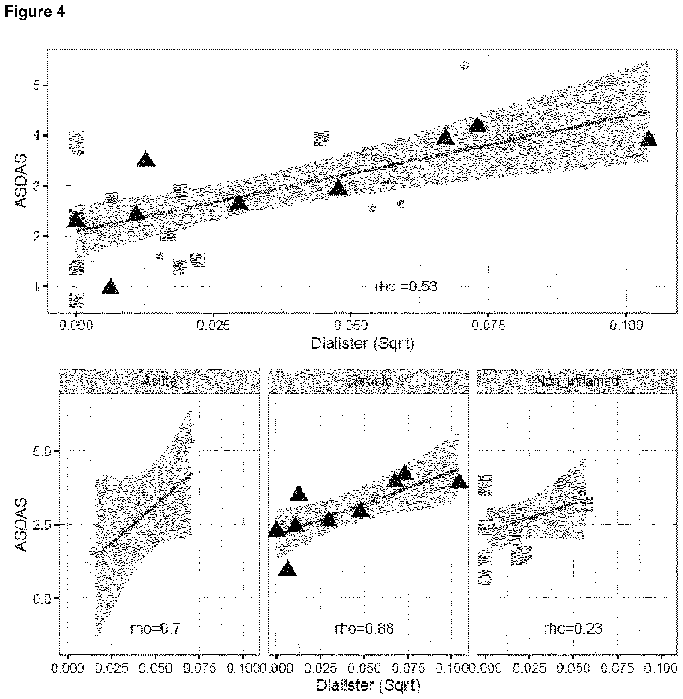

**Figure 4****. Spearman's rank correlation of the abundance of the genus** *Dialister* **with inflammation status in colonic biopsies.** *Dialister* positive correlates with ASDAS values (rho = 0.53, p=0.0038, q=0.1102; Acute (rho = 0.7, p=0.1167, q=0.175), Chronic (rho = 0.88, p=0.0015, q=0.0046) and Non inflamed (rho = 0.23, p=0.2199., q = 0.2199)

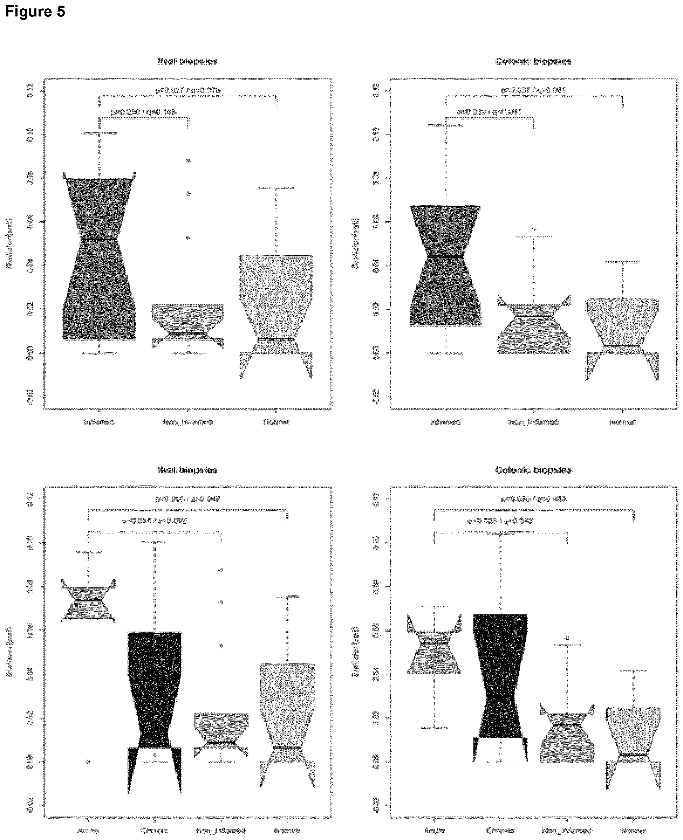

**Figure 5****.** *Dialister* **abundance in** ileal **and colonic biopsies from SpA patients and healthy controls (Normal).** T-test analysis reveals that abundance of *Dialister* is significantly higher in ileal and colonic biopsies from the inflamed versus non-inflamed and healthy control (normal) groups. All these comparisons remain significant after multiple test correction for a FDR <0.10. *Dialister* abundance is presented by square root (Sqrt) to facilitate visualization.

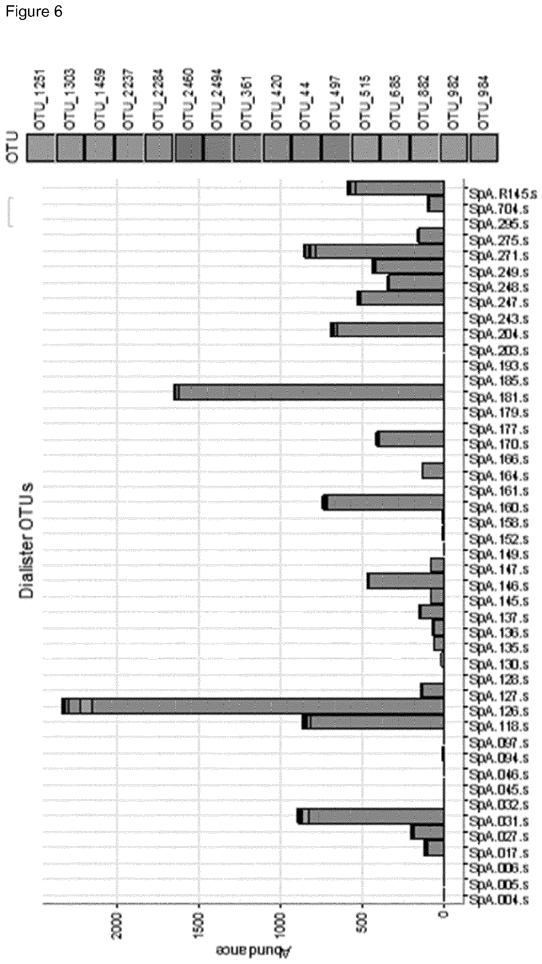

**Figure 6****.** *Dialister* **abundance in stool samples from SpA patients.** *Dialister* is present in 34 out of 46 stool samples from SpA patients whereby OTU_44 is the main contributor.

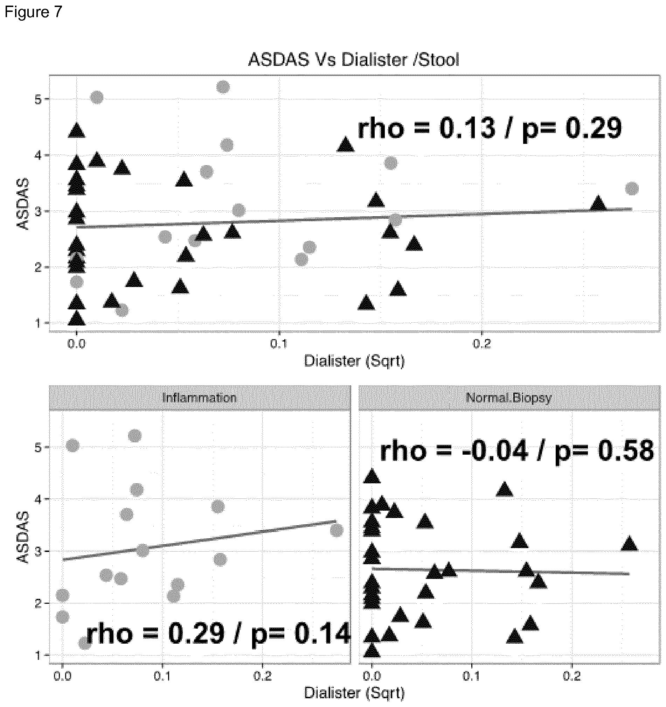

**Figure 7****. Positive** *Dialister* **correlation from stool samples with ASDAS.** The positive correlation value of 0.13 for all stool samples (upper panel) can be divided in 0.29 for patients with inflammation (lower left) and -0.04 for patients without inflammation (lower right).

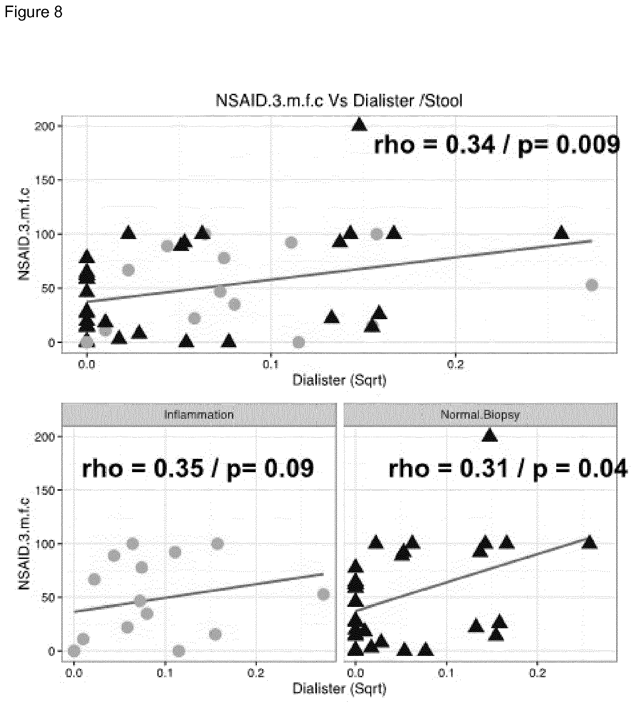

**Figure 8****.** *Dialister* **abundance positively** correlates **with** levels **of intake of non-steroidal anti-inflammatory drugs (NSAID).** The positive correlation value of 0.34 for all stool samples (upper panel) can be divided in 0.35 for patients with inflammation (lower left) and 0.31 for patients without inflammation (lower right).

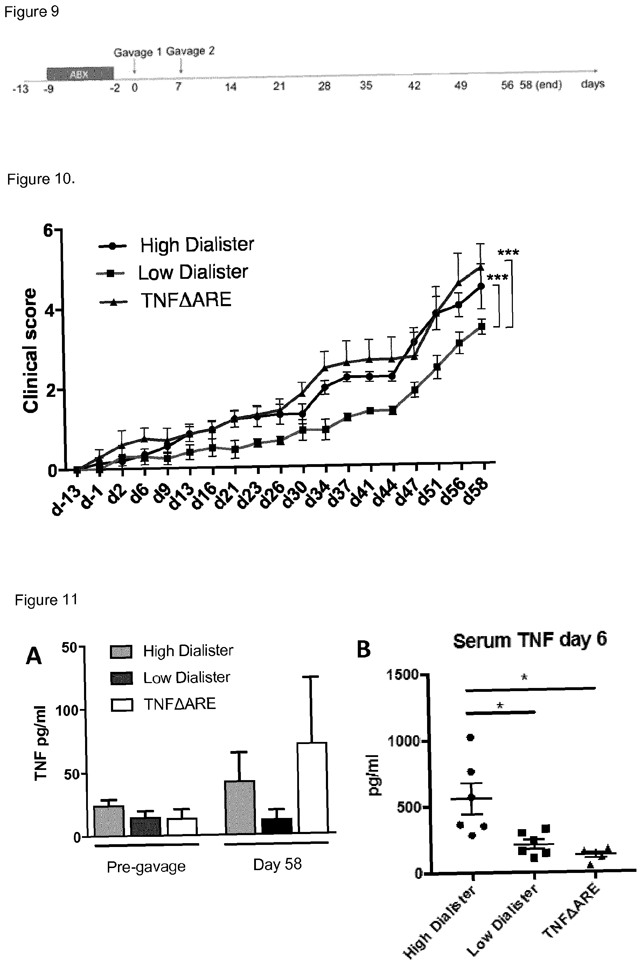

**Figure** 9. **Schematic representation of the timeline of the stool transplant experiment in mice.** Day 0 is the time-point of the first gavage. The antibiotic treatment (ABX) started at day -9 and ended at day -2. A second gavage was performed one week after the first. Mice were followed until day 58.
**Figure 10****. Clinical** scores **of peripheral** arthritis **measured during the course of the stool transplant experiment in female mice.** High Dialister (n=6) and TNFΔARE (n=5) groups develop more severe arthritis compared to the Low Dialister group (n=6): P < 0,001 (mixed models for repeated measures test).
**Figure 11****. Mice that were treated with high Dialister stool samples showed higher TNF levels in their serum.** (A) A clear difference in serum TNF level is observed in the high Dialister and TNFΔARE groups before gavage and at day 58. TNF levels of the low Dialister group remained unchanged. (B) Already at day 6 of the experiment, the serum TNF levels of the high Dialister group was significantly higher than that of the low Dialister group.

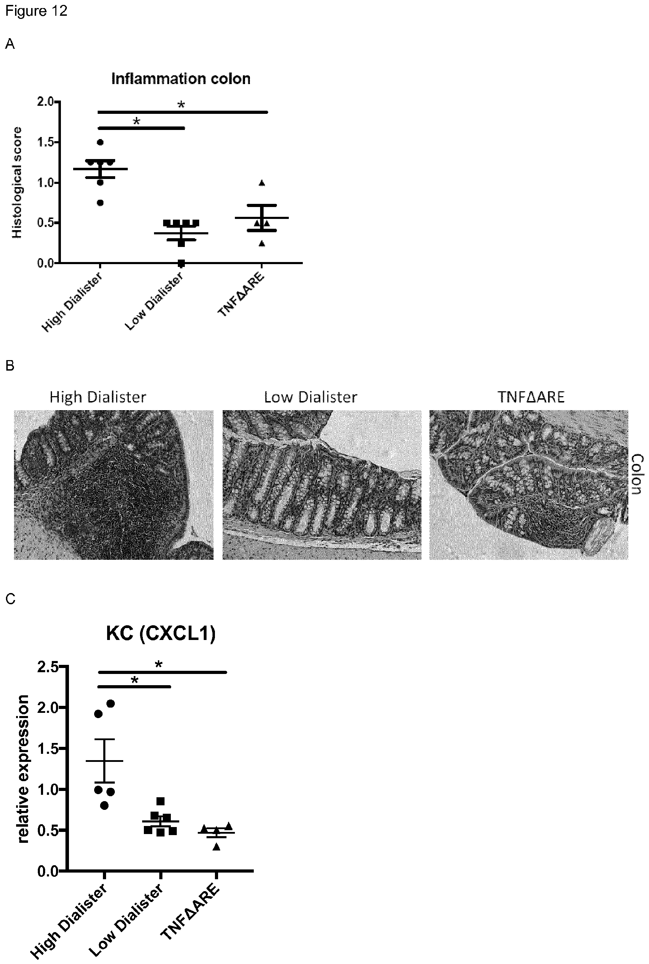

**Figure 12****. Analysis of gut histology and KC expression in the three experimental groups.** (A-B) A highly increased colonic inflammation could be observed in the high Dialister group compared to the low Dialister group and the TNFΔARE group. (A) shows the histological score and (B) shows representative histological sections of colon tissue in the different experimental groups (H&E staining - 20X). (C) RT-QPCR analysis of KC/CXCL1 in colonic tissue revealed a significantly increased expression of the chemokine KC.

### DETAILED DESCRIPTION OF THE INVENTION

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Unless otherwise defined herein, scientific and technical terms and phrases used in connection with the present invention shall have the meanings that are commonly understood by those of ordinary skill in the art. Generally, nomenclatures used in connection with, and techniques of molecular and cellular biology, structural biology, biophysics, pharmacology, genetics and protein and nucleic acid chemistry described herein are those well-known and commonly used in the art. The methods and techniques of the present invention are generally performed according to conventional methods well known in the art and as described in various general and more specific references that are cited and discussed throughout the present specification unless otherwise indicated. See, for example, Sambrook et al. Molecular Cloning: A Laboratory Manual, 3th ed., Cold Spring Harbor Laboratory Press, Cold Spring Harbor, N.Y. (2001); Ausubel et al., Current Protocols in Molecular Biology, Greene Publishing Associates (1992, and Supplements to 2002); Rupp, Biomolecular crystallography: principles, Practice and Applications to Structural Biology, 1st edition, Garland Science, Taylor & Francis Group, LLC, an informa Business, N.Y. (2009); Limbird, Cell Surface Receptors, 3d ed., Springer (2004); Flow Cytometry Protocols, 2nd ed. Humana Press (2004); Antibody engineering, 2nd ed. Springer (2010).

The pathogenesis of spondyloarthritis (SpA) spans several inflammatory arthritis diseases and a systems biology approach was used as described herein to identify novel correlations between spondyloarthritis and gut microflora. For example, the studies disclosed herein successfully demonstrated a link between the composition of the gut microbiome and more particular the composition of the *Dialister* spp. population in SpA patients with and without gut inflammation and the composition of the gut microbiome in healthy subjects. This has led to the development of methods and means to treat, prevent or reduce the severity of inflammation-associated disorders or conditions in a subject in need thereof, such as SpA patients or subjects with related inflammatory diseases.

A *Dialister* spp. modulating composition for use as a medicament is herein disclosed. "Modulating", or grammatically equivalent terms, as used herein refers to affecting or influencing *Dialister* spp. in a subject. As used herein, a *"Dialister* spp. modulating composition" thus refers to any substance or combination of substances that affects or influences *Dialister* spp. and/or the abundance of *Dialister* spp. in a subject. Without the purpose of being limiting this can be achieved by modifying growth and/or proliferation of individual *Dialister* cells, changing the activity of said cells, altering the size of *Dialister* colonies and/or changing presence or residence of *Dialister* spp. in the subject. Non-limiting examples of a *"Dialister* spp. modulating composition" are an antimicrobial agent, such as a peptide or small molecule, or a bacterial strain or a population of bacterial strains, or any combination thereof. Non-limiting examples of antimicrobial agents against *Dialister* spp. are antibiotics (such as amoxicillin, amoxicillin-clavulanate, ticarcillin, ticarcillin-clavulanate, piperacillin, cephalothin, cefoxitin, imipenem, ertapenem, erythromycin, pristinamycin, clindamycin, telithromycin, levofloxacin, ciprofloxacin, moxifloxacin, rifampin, doxycycline, chloramphenicol, metronidazole (Morio et al 2007 Antimicrobial Agents and Chemotherapy 51: 4498-4501) among others), antimicrobial peptides (either synthetic, insect or host derived that kill bacteria by pore formation in bacterial wall and/or membrane or that inhibit cell wall formation or metabolic function or that inhibit macromolecular synthesis or that induce aggregation of bacterial proteins) or other antimicrobial agents including but not limited to engineered bacteriophages, lysins, antibodies.

Also disclosed is the above described *Dialister* spp. modulating composition comprising a population of bacterial strains. Said population can comprise *Dialister* spp. Also disclosed is the above described *Dialister* spp. modulating composition comprising a population of bacterial strains, wherein said population comprises at least one *Dialister* OTU selected from Table 1 or from Table 3. "Modulating" *Dialister* spp. can refer to reducing the abundance of *Dialister* spp. in a subject. The reduction in abundance of *Dialister* spp. which can be achieved by said *Dialister* spp. modulating composition is at least 10%, at least 20%, at least 30%, at least 40%, at least 50%, at least 60%, at least 70%, at least 80%, at least 90%, at least 95%. A 100% reduction is equal to eliminating *Dialister* spp. in a subject below a detectable level. Also disclosed is that said *Dialister* spp. to be reduced in its abundance is *Dialister invisus.* Said *Dialister* spp. can be one of the *Dialister* OTUs selected from Table 4.

The present disclosure also provides a *Dialister* spp. intestinal abundance modulating composition for use as a medicament. A *"Dialister* spp. intestinal abundance modulating composition" as used herein is any substance or combination of substances that affects or influences the abundance of *Dialister* spp. in the gut of a subject, particularly that of *Dialister invisus,* even more particularly one of the *Dialister* OTUs selected from Table 4. "Modulating" the intestinal abundance of *Dialister* spp. can refer to reducing the abundance of *Dialister* spp. in the gut or particularly the abundance of *Dialister invisus* or more particularly one of the *Dialister* OTUs selected from Table 4. This is equivalent as saying that a composition is disclosed reducing the intestinal abundance of *Dialister* spp. for use as a medicament, particularly said *Dialister* spp. is *Dialister invisus,* more particularly said *Dialister* spp. is one of the *Dialister* OTUs selected from Table 4. The reduction in abundance of *Dialister* spp. or *Dialister invisus* in the gut which is achieved by said *Dialister* spp. intestinal abundance modulating composition is at least 10%, at least 20%, at least 30%, at least 40%, at least 50%, at least 60%, at least 70%, at least 80%, at least 90%, at least 95%. A 100% reduction is equal to completely eliminating *Dialister* spp. or *Dialister invisus* in the gut of a subject. As used herein, the term "gut" generally comprises the stomach, the colon, the small intestine, the large intestine, cecum and the rectum. In addition, regions of the gut may be subdivided, e.g. the right versus the left side of the colon may have different microflora populations due to the time required for digesting material to move through the colon, and changes in its composition in time. Synonyms of gut include the "gastrointestinal tract", or possibly the "digestive system", although the latter is generally also understood to comprise the mouth, esophagus, etc. Further non-limiting examples of a *"Dialister* spp. modulating composition" described above also apply here.

Also disclosed herein is a *Dialister* spp. modulating composition for use in treating, preventing or reducing the severity of inflammation in a subject in need thereof. The above described *Dialister* spp. modulating composition can be used in combination with commonly used anti-inflammatory drugs such as inhibitors of cyclooxygenase activity (aspirin, celecoxib, diclofenac, diflunisal, etodolac, ibuprofen, indomethacin, ketoprofen, ketorolac, meloxicam, nabumetone, naproxen, oxaprozin, piroxicam, salsalate, sulindac, tolmetin, among others) or corticosteroids (prednisone, dexamethasone, hydrocortisone, methylprednisolone, among others) or in combination with commonly used analgesics (acetaminophen, duloxetine, paracetamol, among others) or in any combination thereof. Also disclosed is the use of the *Dialister* spp. modulating composition to (re)sensitize patients to anti-inflammatory drugs.

"Inflammation" as used herein can be gut or joint inflammation. Methods to evaluate gut inflammation or joint inflammation are well-known to the person skilled in the art. The most widely used test to measure markers of body-wide inflammation are erythrocyte sedimentation rate and C-reactive protein (CRP). At the macro level and for gut inflammation, such methods include clinical evaluation of the consistency of stool (wherein diarrhea is indicative for gut inflammation), the presence of rectal bleeding, abdominal pain and weight loss. On the micro level, methods to evaluate gut inflammation include histological analyses of stained colon sections, measurements of pro-inflammatory cytokines (such as IL-1beta, IL-6, TNF-alpha), determination of myeloperoxidase activity, fecal excretion of calprotectin (a stable neutrophil specific marker) (Kim et al 2012, J Vis Exp, 60: e3678; Tibble et al 2000, Gut, 47: 506-513). These methods can also be used quantitatively and thus to evaluate the severity of the gut inflammation. A non-limiting example of evaluating joint inflammation is X-ray analysis. By comparing the status or level of inflammation before and after the treatment of the invention, the effect of the treatment can be evaluated. "Reducing the severity of inflammation" or more particular "reducing the severity of gut or joint inflammation" means that the treatment has a positive therapeutic effect on the inflammation status of a patient. The inflammation status of a patient is linked to the term "disease activity score" as used herein and refers to an assessment of inflammation associated disease activity, to determine whether the signs and symptoms have reduced or stopped, and if treatment needs to be adjusted. Progression or worsening of the disease will lead to an increased disease activity score. As used herein, the term "patient" or "individual" or "subject" typically denotes humans, but may also encompass reference to non-human animals, preferably warm-blooded animals, more preferably mammals, such as, e.g. non-human primates, rodents, canines, felines, equines, ovines, porcines, and the like.

Also disclosed herein is a *Dialister* spp. modulating composition for use in treating, preventing or reducing the severity of SpA. The *Dialister* spp. modulating composition is also disclosed for use in treating, preventing or reducing the severity of inflammation in a subject in need thereof, wherein said inflammation is associated with spondyloarthritis and/or inflammatory bowel disease. The *Dialister* spp. modulating composition is also disclosed for use in treating, preventing or reducing the severity of gut inflammation in a subject in need thereof, wherein said gut inflammation is associated with spondyloarthritis or inflammatory bowel disease or a combination thereof. As used herein, the term "spondyloarthritis" or abbreviated "SpA" refers to a group of closely related, but clinically heterogeneous, inflammatory arthritis diseases with common features, including inflammation of the spine, eyes, skin, joints and gastrointestinal tract. This SpA group is also sometimes referred to as spondylitis and spondyloarthropathies. As used herein, SpA includes ankylosing spondylitis (including non-radiographic axial SpA, i.e. ankylosing spondylitis diagnosed using MRI), reactive arthritis, psoriatic arthritis, enteropathic arthritis (arthritis associated with inflammatory bowel disease or IBD related arthritis), undifferentiated spondyloarthritis, juvenile idiopathic arthritis and juvenile-onset SpA. Characteristics of these SpA diseases include inflammatory arthritis of the spine, peripheral arthritis that differs from rheumatoid arthritis, extra articular manifestations of inflammatory bowel disease, arthritis and uveitis, seronegativity for rheumatoid factor and some degree of heritability, including the presence of the gene HLA-B27. It is thus clear that in current disclosure SpA is not rheumatoid arthritis.

As used herein, the term "inflammatory bowel disease" or abbreviated "IBD" refers to an umbrella term for inflammatory conditions of the gut under which both Crohn's disease and ulcerative colitis fall. In people with IBD, the immune system mistakes food, bacteria, or other materials in the gut for foreign substances and responds by sending white blood cells into the lining of the bowels. The result of the immune system's attack is chronic inflammation. Crohn's disease and ulcerative colitis are the most common forms of IBD. Less common IBDs include microscopic colitis, diverticulosis-associated colitis, collagenous colitis, lymphocytic colitis and Behçet's disease. Hence, also disclosed herein is a *Dialister* spp. modulating composition for use in treating, preventing or reducing the severity of inflammation in a subject in need thereof, wherein said inflammation is associated with at least one of the following disorders or conditions: spondyloarthritis, ankylosing spondylitis, reactive arthritis, psoriatic arthritis, enteropathic arthritis, undifferentiated spondyloarthritis, juvenile idiopathic arthritis, inflammatory bowel disease, Crohn's disease, ulcerative colitis and any combination thereof.

Also disclosed is the in any of the above described *Dialister* spp. modulating compositions for use to decrease the symptoms of gut and/or joint inflammation or for use to decrease the symptoms of SpA and/or IBD.

Also disclosed is the *Dialister* spp. modulating compositions above described comprising a compound or a bacterial population. Said bacterial population can comprise *Dialister* spp. The said bacterial population or the bacterial population comprising *Dialister* spp. can be administered to a subject in need thereof as a fecal transplant or as an oral formulation. The "bacterial population comprising *Dialister* spp." can be derived for example, but without the purpose of being limiting, from bacterial collections, after propagation in *in vitro* culturing conditions but can also originate from any subject, particularly from healthy subjects. The bacterial population can consist of a single bacterial OTU or can be a cocktail of two or more bacterial OTUs.

The present disclosure thus also describes *Dialister* spp. obtained from a healthy subject for use as a medicament, more particularly for use in treating, preventing or reducing the severity of SpA or of inflammation in a subject in need thereof. The *Dialister* spp*.* obtained from a healthy subject can be one or more *Dialister* strains or OTUs. Said one or more OTUs can be selected from Table 1 or from Table 3, while said inflammation can be gut and/or joint inflammation or associated with SpA or IBD or a combination thereof.

To "obtain" *Dialister* bacteria from a subject the *Dialister* spp. can be directly isolated from any subject's biological sample. Non-limiting examples of said specimen or sample are dental specimens from tooth cavities, blood samples, nasopharyneal secretions, mucosal biopsy sample, stool sample, a sample of the lumen conten, an oral sample, a urine sample, a vaginal sample (Downes et al 2003, Int J Syst Evol Microbiol, 53: 1937-1940; Rôças and Siqueira 2006, Journal of endodontics, 32: 1057-1061; Morio et al 2007, Antimicrobial Agents and Chemotherapy, 51: 4498-4501; Olitsky and Gates 1921, JEM, 33: 125-145). Said specimen can be obtained or isolated from fecal material of a subject. The herein described *Dialister* spp. for use as a medicament can also be derived of any culturing or other process that results in or is intended to result in replication of the *Dialister* population after obtaining any of the above samples. The *Dialister* spp. for use as a medicament can also be derived from *Dialister* bacteria obtained from collections. A non-limiting example of said collections are bacterial collections that are obtained from fecal samples and/or of microbiota obtained from intestinal biopsies or other clinical biopsies. "OTU" (or plural "OTUs") as abbreviation of "operational taxonomic unit" as used herein refers to a terminal leaf in a phylogenetic tree and is defined by a nucleic acid sequence that shares sequence identity to nucleic acid sequences at the level of species. Standardly, the 16S sequence or a portion of the 16S sequence is used for this purpose but any other sequence or the entire genome can be used (e.g. a functionally conserved housekeeping gene found broadly across the eubacterial kingdom). In microbiology, "16S sequencing" or "16S" refers to sequence derived by characterizing the nucleotides that comprise the 16S ribosomal RNA gene(s).The bacterial 16S rRNA is approximately 1500 nucleotides in length and is used in reconstructing the evolutionary relationships and sequence similarity of one bacterial isolated to another using phylogenetic approaches. A "type" or a plurality of "types" of bacteria includes an OTU or a plurality of different OTUs, and also encompasses a strain, species, genus, family or order of bacteria. OTUs share at least 95%, 96%, 97%, 98%, or 99% sequence identity. OTUs are frequently defined by comparing sequences between organisms. Sequences with less than 95% sequence identity are not considered to form part of the same OTU.

Also disclosed herein is that said *Dialister* spp. modulating composition can be a composition reducing the intestinal abundance of *Dialister* spp. Said *Dialister* spp. modulating compositions can be a composition modulating *D. invisus* or more particularly modulating the intestinal abundance of *D. invisus* while said composition reducing the intestinal abundance of *Dialister* spp. can be composition reducing the intestinal abundance of *D. invisus.*

Current disclosure also describes a composition for use in treating, preventing or reducing the severity of SpA or of inflammation in a subject in need thereof, said composition comprising a bacterial population comprising *Dialister* spp. obtained from a subject. The composition can be a fecal transplant. The composition can be an oral formulation. Said inflammation can be gut inflammation or joint inflammation. Also disclosed is a composition for use in treating, preventing or reducing the severity of SpA or of inflammation in a subject in need thereof, said composition comprising a bacterial population comprising *Dialister* spp. obtained from a healthy subject and said inflammation is associated with at least one of the following disorders or conditions: spondyloarthritis, ankylosing spondylitis, reactive arthritis, psoriatic arthritis, enteropathic arthritis, undifferentiated spondyloarthritis, juvenile idiopathic arthritis, inflammatory bowel disease, Crohn's disease, ulcerative colitis and any combination thereof. The above described subject can be a healthy subject.

Also described is a composition for use in treating, preventing or reducing the severity of SpA or of inflammation in a subject in need thereof, said composition comprising an extract or preparation of feces of a subject, said extract or preparation comprising human gut, colon or intestinal flora, wherein said flora comprises at least *Dialister* spp. Said inflammation can be gut inflammation or joint inflammation. Said inflammation can be associated with SpA or IBD or a combination thereof. The above described subject can be a healthy subject.

The compositions as described herein can be administered to the subject in need thereof as a fecal transplant, as an oral formulation, as a pharmaceutical preparation, as a medical food preparation, a nutritional or dietary supplement preparation, as a food product or food additive, as a beverage product or a beverage additive. Depending on the formulation, the composition can be administered rectally, orally, topically, or by nasal administration or respiratory administration. The composition can also comprise a second bacterial population, wherein a second bacterial population is any bacterium other than *Dialister* spp.

The compositions as described herein may also comprise at least 1×10⁴, 1×10⁵, 1×10⁶, 1×10⁷, 1×10⁸, 1×10⁹, 1×10¹⁰, or greater than 1×10¹⁰ colony forming units (CFUs) of viable *Dialister* spp. and said *Dialister* spp. are capable of functionally populating the gut of the subject. "Functional populating" or "colonization" of a host organism's gut includes the non-transitory residence of a bacterium, in this case *Dialister* spp.

Also disclosed is a composition for use in reducing the intestinal abundance of disease associated *Dialister* spp. in SpA patients and/or in IBD patients and/or in patients suffering from inflammation, wherein said composition comprises an extract or preparation of feces of a subject, in particular a healthy subject, wherein said extract or preparation comprising human gut, colon or intestinal flora and wherein said flora comprises one or more non-disease associated *Dialister* spp. Said inflammation can be gut inflammation or joint inflammation.

The term "disease associated" is used herein in reference to *Dialister* bacteria and refers to a bacterial strain from the *Dialister* genus that is capable of causing or affecting a disease, disorder or condition of a host organism containing the disease associated strain. More particularly, disease associated *Dialister* strains can cause gut and/or joint inflammation. "Disease associated *Dialister* spp." as used herein is equivalent to one *Dialister* OTU or a population of native enteric *Dialister* OTU(s) in patients suffering from or at risk of developing gut and/or joint inflammation in SpA patients or in subjects with ankylosing spondylitis, reactive arthritis, psoriatic arthritis, enteropathic arthritis, undifferentiated spondyloarthritis, juvenile idiopathic arthritis, inflammatory bowel disease, Crohn's disease, ulcerative colitis and any combination thereof. Said disease associated *Dialister* spp. can be *Dialister invisus* or one of the OTU's selected from Table 4. "Non-disease associated *Dialister* spp." in the context of this disclosure are *Dialister* spp. that are not associated with inflammation and that can for example be collected or obtained from healthy subjects, or elsewhere.

Intestinal abundance of bacteria can be measured in stool samples of subjects, but also more invasive methods (e.g. biopsies) are known by the person skilled in the art. The reduction in intestinal abundance of disease associated *Dialister* spp. which is achieved by said composition is at least 10%, at least 20%, at least 30%, at least 40%, at least 50%, at least 60%, at least 70%, at least 80%, at least 90%, at least 95%. A 100% reduction is equal to completely removing disease associated *Dialister* spp. in a subject. Also disclosed is a composition for replacing disease associated *Dialister* spp. in a patient suffering from or at risk of developing SpA or inflammation wherein said composition comprises an extract or preparation of feces of a subject, wherein said extract or preparation comprising human gut, colon or intestinal flora and wherein said flora comprises one or more non-disease associated *Dialister* spp. Said inflammation can be gut inflammation or joint inflammation and said subject can be a healthy subject. "Reducing abundance" or "reducing colonization" of a host subject's gut by a bacterium includes a reduction in the residence time of the bacterium in the gut as well as a reduction in the number or concentration of the bacterium in the gut or adhered to the luminal surface of the gut. Measuring reduction of adherent bacteria may be demonstrated directly e.g. by a biopsy sample, or indirectly e.g. by measuring the disease associated burden in the stool of a subject. The above described *Dialister* spp. modulating compositions can be compositions that modulate *D. invisus* or reduce the intestinal abundance of *D. invisus.*

Also disclosed are methods for treating, preventing or reducing the severity of SpA or of inflammation in a subject in need thereof, said method comprising administering to said subject an effective amount of a composition modulating the intestinal abundance of *Dialister* spp. in said subject. Said inflammation can be gut and/or joint inflammation. Said inflammation can be associated with at least one of the following disorders or conditions: spondyloarthritis, ankylosing spondylitis, reactive arthritis, psoriatic arthritis, enteropathic arthritis, undifferentiated spondyloarthritis, juvenile idiopathic arthritis, inflammatory bowel disease, Crohn's disease, ulcerative colitis and any combination thereof. Said composition can be a composition reducing the intestinal abundance of *Dialister* spp. or *D. invisus* in said subject. Said composition modulating, affecting, influencing or reducing the intestinal abundance of *Dialister* spp. or *D. invisus* can comprise a bacterial population which includes at least *Dialister* spp. Said bacterial population can comprise non-disease associated *Dialister* spp. derived from healthy subjects. Said bacterial population can also comprise a *Dialister* OTU selected from Table 1 or from Table 3. The bacterial population can be administered for example by fecal transplantation or as an oral formulation. The above-mentioned methods aim at modulating the *Dialister* spp. that are associated with disease in subjects that suffer from or at risk of developing SpA or IBD or a combination thereof. These disease associated *Dialister* spp. can comprise *Dialister invisus.*

Also disclosed are methods of treating, preventing or reducing the severity of SpA or of inflammation in a subject in need thereof, said method comprising administering to said subject an effective amount of a composition comprising a bacterial population, wherein said bacterial population comprises *Dialister* spp. obtained from a healthy subject. Said inflammation can be gut and/or joint inflammation. Said composition can be a fecal transplant or an oral formulation. It is described that the administration of an effective amount of a composition comprising *Dialister* spp. or more particularly non-disease associated *Dialister* spp. can decrease the intestinal abundance of disease associated *Dialister* spp. in a subject suffering from or at risk of developing gut and/or joint inflammation. A non-limiting example of such a disease associated *Dialister* spp. is *Dialister invisus.* The administration of an effective amount of a composition comprising *Dialister* spp. can also increase the intestinal abundance of non-disease associated *Dialister* spp*.* in a subject suffering from or at risk of developing gut and/or joint inflammation. Said inflammation can be associated with SpA or IBD or a combination thereof.

An "effective amount" of a composition is equivalent to the dosage of the composition that leads to treatment, prevention or a reduction of the severity of inflammation status in a patient. Said inflammation can be gut and/or joint inflammation for which several methods are known to the person skilled in the art to evaluate or thus to diagnose the severity of the inflammation.

Also disclosed are methods to reduce the intestinal abundance of *Dialister* spp. in a subject suffering from or at risk of developing gut and/or joint inflammation, wherein said *Dialister* spp. are causative to said gut and/or joint inflammation, said method comprising performing a fecal microbiota transplantation from a healthy subject to a subject with gut and/or joint inflammation. Also described are methods to replace *Dialister* spp. in a SpA patient or IBD patient or in subjects suffering from or at risk of developing gut and/or joint inflammation, by *Dialister* spp. isolated from a healthy subject, said method comprising performing a fecal microbiota transplantation from a healthy subject to a subject with gut and/or joint inflammation. Also described are methods to replace *Dialister* spp. in a SpA patient or in a IBD patient or in subjects suffering from or at risk of developing gut and/or joint inflammation by *Dialister* spp. isolated from a healthy subject, said method comprises administering a composition to said SpA patient or patient suffering from gut and/or joint inflammation, wherein said composition comprises an extract or preparation of feces of said healthy subject, wherein said extract or preparation comprises human gut, colon or intestinal flora, and wherein said flora comprises at least *Dialister* spp. Said *Dialister* spp. isolated from a healthy subject can be one or more *Dialister* OTUs selected from Table 1 or from Table 3.

Also disclosed are screening methods to identify a compound that modulates intestinal abundance of *Dialister* spp. in a subject suffering from or at risk of developing SpA or inflammation, said method comprising the following steps:
- contacting isolated *Dialister* spp. with a compound in an *in vitro* environment;
- selecting a compound that modulates growth of *Dialister* spp.;
to identify a compound that modulates the intestinal abundance of *Dialister* spp. in a subject in need thereof.

Said inflammation can be gut and/or joint inflammation. Said inflammation can also be associated with SpA or IBD or a combination thereof.

Also described are screening methods to identify a compound that reduces intestinal abundance of *Dialister* spp. in a subject suffering from or at risk of developing SpA or gut and/or joint inflammation, said method comprising the following steps:
- contacting isolated *Dialister* spp. with a compound in an *in vitro* environment;
- selecting a compound that inhibits growth of *Dialister* spp.;
to identify a compound that reduces the intestinal abundance of *Dialister* spp. in a subject in need thereof. Also disclosed are screening methods to identify a compound that selectively reduces the intestinal abundance of disease associated *Dialister* spp., said method comprising the following steps:
- contacting isolated disease associated *Dialister* spp. from subjects suffering from or at risk of SpA or of gut and/or joint inflammation and isolated non-disease associated *Dialister* spp. from healthy controls with a compound in an *in vitro* environment;
- selecting a compound that inhibits growth of at least one of said disease associated *Dialister* spp.;
to identify a compound that selectively reduces the intestinal abundance of disease associated *Dialister* spp. in a subject in need thereof.

Inhibition of bacterial growth may include inhibiting the increase in size and/or number of the bacterium and/or inhibiting the proliferation or multiplication of the bacterium. A person skilled in the art is very familiar with assays that evaluate inhibition of bacterial growth e.g. on solid bacterial growth media this can be done by evaluating the size and/or number of colonies, while in liquid bacterial growth medium the optical density of a bacterial sample can be measured at a wavelength of 600 nm (i.e. OD600 measurement which are known by the skilled one). Inhibition of colonization of a disease associated bacterium may be demonstrated by measuring the amount or burden of a disease associated bacterium before and after a treatment. An inhibition of the act of inhibiting includes the total cessation and partial reduction of one or more activities of a bacterium, such a growth, proliferation, colonization and function. The function of the compound can be cytotoxic or cytostatic for the bacterium. Cytotoxic means the ability to kill a bacterial cell, while cytostatic means the ability to inhibit, partially or fully, growth, metabolism and/or proliferation of a bacterial cell.

Also described are methods to produce a pharmaceutical composition comprising a compound derived from a screening method, said screening method comprising the following steps:
- contacting isolated *Dialister* spp. with a compound in an *in vitro* environment;
- selecting a compound that modulates growth of *Dialister* spp.;
to identify a compound that modulates the intestinal abundance of *Dialister* spp. in a subject in need thereof.

Also described are methods to produce a pharmaceutical composition comprising a compound derived from a screening method, said screening method comprising the following steps:
- contacting isolated disease associated *Dialister* spp. from subjects suffering from or at risk of SpA or of gut and/or joint inflammation and isolated non-disease associated *Dialister* spp. from healthy controls with a compound in an *in vitro* environment;
- selecting a compound that inhibits growth of at least one of said disease associated *Dialister* spp.;
to identify a compound that selectively reduces the intestinal abundance of disease associated *Dialister* spp. in a subject in need thereof.

It is further described that the *Dialister* spp. modulating composition of this disclosure or the herein described compound that modulates growth of *Dialister* spp. is a composition or compound that specifically modulates *Dialister* spp. Said composition or said compound can be a composition or compound that selectively modulates disease associated *Dialister* spp. Said disease associated *Dialister* spp. can comprise *Dialister invisus* or even more particularly can comprise one of the OTU's selected from Table 4.

The following examples are intended to promote a further understanding of the invention. While the invention is described herein with reference to illustrated embodiments, it should be understood that the invention is not limited hereto. Those having ordinary skill in the art and access to the teachings herein will recognize additional modifications and embodiments within the scope thereof. Therefore, the invention is limited only by the claims attached herein.

### EXAMPLES

### Example 1. Microbial composition and diversity across biopsy samples

We first assessed overall variation of microbial composition and diversity across mucosal samples from 27 SpA patients and 15 healthy controls. SpA patient characteristics at baseline according to bowel histology are summarized in Table 2. Consistent with earlier reports, chronic inflammation was associated with younger age and male sex (p= 0.010 and p = 0.021, respectively). None of the other clinical characteristics (BMI, symptom duration, CRP, BASDAI, ASDAS, HLAB27, AS versus non-radiographic axial SpA, smoking status) were significantly associated with bowel histology. There was also no significant difference in NSAID index. Microbial diversity comprises richness (observed species; here measured by observed OTU richness, Figure 1a) and evenness (how the relative abundance or biomass is distributed among species); these combined properties are measured by the Shannon Diversity index, Figure 1b. Whole sample community composition-based ordination on Bray Curtis distances did not show clear sample separation based on biopsy location (Figure 1c). Likewise no significant differences in richness and evenness of colonic and ileal biopsies were detected. Overall, bacterial profiles of ileum and colon were more similar within one individual than same-site samples across persons. However, all further analyses were performed on either ileal or colonic samples, using only one biopsy sample per patient. Given the overall low bacterial yield from biopsy samples, we next assessed any interference from contaminant bacteria. To this aim, we analysed the bacterial profile of negative controls (blanks; see methods). Negative controls exhibit a profile dominated by genera belonging to the phylum Proteobacteria, which represents a minor contributor to the profile of our biopsies. The profile of gut microbiota reported in this study is consistent with the general profile of the human gut microbiome, dominated by Firmicutes and Bacteroidetes. These results rule out any major contamination event during the generation of molecular data.

### Example 2. Dialister, a microbial marker of disease activity in SpA patients

A trend towards greater microbial richness was observed in inflamed versus non-inflamed biopsies with higher richness in chronically inflamed versus acute or non-inflamed biopsies (Figure 2, results are shown for ileal biopsies). Inflamed biopsies also presented higher phylogenetic diversity (PD) compared to non-inflamed and normal (healthy control) samples. A positive association trend between the type of inflammation and microbial evenness (Shannon index) was found, with higher evenness in acute, followed by chronic inflammation and lower levels in non-inflamed and healthy control biopsies, although this observation was not significant (Figure 2).

We next explored the relationship between bacterial composition and disease activity parameters. We observed a positive correlation between the abundance of the genus *Dialister* and ASDAS (rho=0.62, FDR-corrected q-value of 0.0096) (Figure 3) as well as BASDAI (rho=0.89, FDR-corrected q-value of 0.062) (data not shown). Results were consistent between acute and chronic inflammation (Figure 3). A consistent correlation was observed in colonic biopsies (rho = 0.53) (Figure 4), which was expected, given the high concordance between *Dialister* abundances in colonic and ileal samples (data not shown). (Nonparametric) t-test analysis revealed that abundance of *Dialister* was significantly higher in ileal and colonic biopsies from the inflamed versus non-inflamed and healthy control groups (FDR < 0.10), with higher abundance seen in acutely inflamed biopsies (Figure 5).

### Example 3. Dialister OTU distribution in stool samples of SpA patients

Following up on the *Dialister* results regarding colonic and ileal mucosa, we examined stool samples from SpA patients with different levels of ASDAS (Ankylosing Spondylitis Disease Activity Score), NSAID (Nonsteroidal anti-inflammatory drug), BASDAI (Bath Ankylosing Spondylitis Disease Activity Index), Inflammation activity and fecal calprotectin. Characteristics of the SpA patients are summarized in Table 5. Bacteria profiling from 46 stools samples was characterized using the methods described in this application. Sequences were quality trimmed, demultiplexed, analyzed and taxonomy identified against RDP/Greengenes database using LotuS. All reads per sample were used for *Dialister* abundance analysis. The rest of the analysis was performed using R libraries on data rarefied at 10000 observations/reads per sample. Using all reads, *Dialister* is present in 74% of the stools samples from SpA patients (35 of 46 patients), with an abundance ranging from 3.3E-17 to 7.7% (Figure 6). From the 16 different OTUs assigned to the genus *Dialister,* OTU 44 is the main contributor, followed by OTU 515, OTU 420 and OTU 685. Similar to the most abundant OTU in biopsy samples (see Example 4), OTU 44 exhibits a sequence match of 100% to *Dialister invisus.* The other OTUs cannot be reliably assigned to a known *Dialister* species based on what is available so far in NCBI.

### Example 4. Correlation between Dialister abundance in stool samples and ASDAS

Besides the analysis of biopsies also stool sample were investigated. The studied stool samples (n=46 stool samples from patients with SpA) exhibit a positive correlation between *Dialister* and ASDAS. This correlation is improved when samples are grouped by gut inflammation status (normal vs. inflamed biopsies). The positive correlation value of 0.13 for all stool samples can be divided in 0.29 for patients with inflammation and -0.04 for patients without inflammation. This pattern is consistent with our results of *Dialister* abundance in colonic and ileal mucosa, but it is not significant (Figure 7). The correlation is driven by OTU 44, which is the most abundant OTU assigned to the *Dialister* genus (Figure 6).

### Example 5. Correlation between Dialister abundance in stool samples and NSAID

*Dialister* abundance positively correlates with levels of intake of non-steroidal anti-inflammatory drugs (Figure 8, rho=0.34, p< 0.01), thus further building evidence for a clear link between *Dialister* abundance and SpA. In conclusion, the data presented here clearly show that the positive correlation between *Dialister* abundance and SpA can also be observed in stool samples. These surprising findings are of great importance for the diagnosis and treatment of SpA and other inflammatory arthritis diseases, since these data show that inflammatory arthritis disease are linked with a high abundance of *Dialister* in the gut of the patients.

### Example 6. Specific Dialister OTUs linked to SpA

Interestingly, it has recently been shown that *Dialister* shows a bimodal abundance distribution across human fecal microbiomes (Lahti et al 2014 Nature Communication 5: 4344 doi:10.1038/ncomms5344), characterized by low- and high-abundant *Dialister* microbiomes, respectively. To further understand the *Dialister* microbiome composition, we performed a phylogenetic study of several *Dialister* OTUs (operational taxonomic units) which are high-abundant in biopsy samples of SpA patients or in stool samples of persons that volunteered in the Flemish Gut Flora project (FGFP). More precisely, a phylogenetic analysis was performed using the maximum likelihood method based on partial 16S rRNA gene sequences encompassing 250 positions corresponding to the V4-16S rDNA region, thus covering app. 15% of the 16S rRNA gene. The FGFP is a large-scale cross-sectional fecal sampling effort in a confined geographical region, more precisely Flanders (Belgium). The FGFP cohort is expected to be representative for the average gut microbiota composition in a Western European population (Falony et al 2016 Science 352: 560-564).

Surprisingly, a clear difference was found between SpA samples and FGFP samples. OTU60 and OTU62 were the most abundant *Dialister* OTUs in SpA patients. Both OTUs had a 100% identical nucleotide sequence (based on 250 positions of V4-16S rDNA) to *D. invisus.* The most abundant *Dialister* OTUs in FGFP samples were OTU4552 (comprising SEQ ID No. 11) that shows highest similarity to *D. micraerophilus,* OTU6938 (comprising SEQ ID No. 12) that shows highest similarity to *D. succinatiphilus,* OTU1378 (comprising SEQ ID No. 13) that does not show high similarity to a known *Dialister* species, and OTU605 (comprising SEQ ID No. 14) and OTU233 (comprising SEQ ID No. 15) that both show highest similarity to *D. invisus.* However, in contrast to OTU60 and OTU62 from SpA patients, the 16S rRNA nucleotide sequence of OTU605 and OTU233 from FGFP samples is not identical to that from *D. invisus.* It thus appears that, at a finer level, both low- and high-abundant *Dialister* populations also harbor different *Dialister* genotypes which rarely coexist in the same microbiome. An overview of the *Dialister* OTU's is given in Tables 1, 3-4. Taken together, with the positive correlation between SpA disease activity and *Dialister* abundance in SpA patients (Example 2) the here reported *Dialister* distribution patterns form the basis for the design of replacement and transfer strategies in SpA patients as described in Examples 7-9.

### Example 7. Replacement therapy with Dialister strains from non-SpA subjects

The results described in Example 6 show that SpA patients differ from the subjects that participated in the FGFP concerning the most-abundant *Dialister* OTUs in their gut microbiome. While *D. invisus* was the most prominent *Dialister* species in SpA patients, the FGFP samples contained at least 4 other high-abundant *Dialister* OTUs. Therefore, we questioned whether a replacement therapy of *Dialister* in SpA patients using *Dialister* strains from non-SpA subjects affects gut inflammation and SpA-related disease characteristics. Replacement therapy aims at outcompeting the endogenous *Dialister* spp. with an exogenous supplementation of *Dialister* spp. isolated from healthy subjects. The results will be the replacement of *Dialister* spp. in the gut of SpA patients by non-disease associated *Dialister* spp. Proof-of-concept for gut microbiome replacement has been shown by Li et al 2016 (Science 352: 586-589). To perform the desired *Dialister* replacement, we make use of both control mice and mice suffering from SpA (TNFΔARE as well the SKG model). For sake of clarity and simplicity, the *Dialister spp.* collected from SpA subjects are referred to as *"SpA-Dialister"* or as "disease associated *Dialister"* while the *Dialister* spp. collected from healthy subjects are referred to as "healthy-*Dialister"*. First, stool samples of healthy mice are collected and subsequently cultured. Next, in mice with active SpA and high fecal SpA-*Dialister,* we administer a cocktail of healthy-*Dialister* strains isolated from stool samples of healthy mice. During therapy, a minimal dose of 10E6 CFU/g of healthy-*Dialister* cocktail encapsulated in gastro-resistant capsules is administered daily for a period of 2 weeks. SpA-related disease activity is monitored.

### Example 8. Fecal transplant study

Next to selectively replacing *Dialister* spp. in SpA subjects, we perform fecal microbial transfer using healthy donors. The major aim here is to remove the dysbiotic microbiota associated with SpA disease and replace it by a more balanced ('normobiotic') gut community from donors without SpA diagnosis. After bowel preparation using a PEG solution, mice with SpA (TNFΔARE or SKG model) receive a fecal suspension of donor stool via a rectal route in three times. The fecal donor suspension contains a total of 200 grams of spontaneous stools from healthy donors collected during the 48h before the procedure (and kept at 4°C) that is homogenized with 400 mL sterile saline. To monitor the targeted alteration of the dysbiotic microbiota associated with SpA disease, stool samples of transplanted mice are collected for 16S rRNA sequencing at baseline, subsequently daily during the first week and then weekly for 8 weeks after fecal transfer. The donor sample that is used for the fecal transfer is analyzed as well. This enables us to further specify microbial stool features of dysbiotic microbiota associated with SpA that will benefit from fecal microbiota transplant and/or donor features that are associated to improvement of SpA symptoms.

### Example 9: Inoculation of antibiotics treated mice with disease associated Dialister spp.

Next, we wanted to demonstrate a pathogenic role for the spondyloarthritis (SpA)-associated bacterial genus *Dialister* during SpA development in mice, by colonizing young TNFΔARE mice with stool samples from human SpA patients containing either high or low concentrations of *Dialister* bacteria, and monitor disease progression overtime.

First, TNFΔARE mice of 6 weeks old were selected and were treated with a broad-spectrum antibiotics cocktail for one week to deplete the resident microbiome and to facilitate colonization of the donor microbiome. Two days after the end of the antibiotics treatment, mice received the donor microbiome (1,2 x10E12 CFU bacteria) by oral gavage. Mice received human stool samples from SpA patients with high *Dialister* abundance or with low *Dialister* abundance or received a TNFΔARE mice stool sample. One week later, a second gavage was given in a similar procedure (Figure 9).

Prior to the oral gavages, the relative abundance of *Dialister* spp. was determined in human stool samples from SpA patients using qPCR. A qPCR assay was performed using KAPA SYBR FAST qPCR Master Mix (2X) / low ROX and the primers Bac-Uni-F (5'-CCATGAAGTCGGAATCGCTAG-3') and Bac-Uni-R (5'-GCTTGACGGGCGGTGT-3') for Bacteria (Ramseier et al, 2009 Journal of Periodontology 80.3: 436-446) and the primers DpneF (5'-GAGGGGTTTGCGACTGATTA-3') and DpneR (5'-CCGTCAGACTTTCGTCCATT-3') for *Dialister* spp. (Nonnenmacher et al, 2004 Journal of microbiological methods 59.1: 117-125). Compared to the general bacterial abundance, the *Dialister* relative abundance was 2.5% for the low *Dialister* sample and 12.0% for the high *Dialister* sample. These two stool samples were used to prepare the inoculum for the first and second mice gavage.

Three experimental groups were thus evaluated:
1. TNFΔARE mice inoculated with a SpA patient stool sample with high *Dialister* content (12% confirmed by Q-PCR,), n=12 (6♂, 6♀)
2. TNFΔARE mice inoculated with a SpA patient stool sample with low *Dialister* content (2,55% confirmed by Q-PCR,), n=12 (6♂, 6♀)
3. TNFΔARE mice inoculated with TNFΔARE mouse stool, n=10 (5♂, 59)

Mice were scored for macroscopic arthritis symptoms twice a week (Jacques et al., Arthritis and Rheumatism, 2010) and blood was collected once a week. At the end of the experiment, mice were euthanized for analysis. The analyses comprised joint pathology (ankle, spine, sacroiliac joint, tail), intestinal pathology (ileum and colon histology), serum analysis for cytokine analysis and qPCR analysis on intestinal tissue.

In the three experimental groups, peripheral arthritis was monitored by macroscopic examination according to Jacques et al., (2010, Arthritis and Rheumatism). Figure 10 convincingly shows that arthritis development (clinical score) is statistically more severe in female mice gavaged with a high *Dialister* containing human SpA stool sample and in female mice gavaged with mouse TNFΔARE stool, compared to female mice gavaged with a low *Dialister* containing human SpA stool sample. High *Dialister* samples thus clearly have pathogenic properties for SpA and joint inflammation associated with SpA and Figure 10 shows that high *Dialister* human SpA stool sample is as pathogenic as TNFΔARE stool. The latter was already demonstrated to be pathogenic in the context of ileal inflammation (Schaubeck et al., Gut 2016). In addition, the low *Dialister* sample has a clear protective property again SpA and joint inflammation associated with SpA since the clinical disease scores of mice treated with the low *Dialister* sample have statistically significant lower scores compared to control TNFΔARE controls (Figure 10).

Fully in line with the above observations, both high *Dialister* and TNFΔARE groups have increased serum TNF at the end of the experiment compared to serum TNF levels before gavage, while serum levels in the low *Dialister* group remains stable throughout the experiment (Figure 11 A). A significantly higher TNF level in the high *Dialister* group compared to the two other experimental groups could already be observed at day 6 of the experiment (Figure 11 B).

Furthermore, on histology, a clear increased colonic inflammation was observed in the high *Dialister* group as shown by increased immune cell infiltration in the lamina propria, and a higher histological score (Figure 12 A-B). Finally, the RNA level of the chemokine KC (or CXCL1) was determined in colonic tissue of the treated animals. KC or CXCL1 is the mice orthologue of human IL-8. RNA was isolated from tissue using Rneasy mini kit (Qiagen 74104), cDNA was generated using iScript reverse transcription kit (Bio-Rad 1708840) and RT-QPCR was performed using a Lightcycler 480 (Roche) with Sensimix sybr no-rox kit (GC Biotech QT650-05). To amplify the murine KC/CXCL1 transcripts the following primers were used: murine KC/CXCL1 fwd: 5'-GAGCCTCTAACCAGTTCCAG-3' and rvs: 5'-TGAGTGTGGCTATGACTTCG-3'. As reference gene the murine SDHA (Succinate Dehydrogenase subunit A) was used (fwd primer: 5'-CGGGCAGGCTCATCGGTGTT-3' and rvs: 5'-TTCGCCCGTAGCCCCCAGTA-3'). In agreement with the above, expression of the chemokine KC (CXCL1) in colonic tissue was significantly higher in the high *Dialister* group compared to the low *Dialister* group (Figure 12 C).

### METHODS TO THE EXAMPLES

### Study Population and Sample Collection

Mucosal samples from 27 SpA patients (Table 2) and 15 healthy controls were used. This study was conducted after approval by the ethical committee of Ghent University Hospital and written informed consent was obtained from all patients. Patients with overt IBD were excluded from this analysis, as were patients with prior exposure to biologic therapy, or use of conventional DMARDs (sulfasalazine, methotrexate, leflunomide), corticosteroids or antibiotics 2 months prior to ileocolonoscopy. NSAID intake 2 months prior to the ileocolonoscopy was estimated by calculation of the NSAID index score, as recommended by ASAS (¹. Ileal and colonic biopsies were classified as normal or inflamed (acute/chronic) by an experienced pathologist. Biopsies used for nucleic acid extraction were kept at -80 °C in sterile cryovials.

### Nucleic acid extraction

A total of 54 biopsy samples, with associated clinical information, (27 colonic and 27 ileal) from SpA patients and 21 biopsy samples from healthy controls (15 ileal and 6 colonic) were used for this study (Table 2). DNA was extracted from biopsies using the PowerMicrobiome RNA Isolation Kit (MO BIO Laboratories Inc., Carlsbad, CA) following manufacturer's instructions, with the addition of 10 minutes at 90 °C before vortexing and with the exclusion of the DNase I step. No more than 11 samples were prepared at the time and each set of extractions included a negative control (blank). All steps were performed under a biohazard type II cabinet and all material was decontaminated using UVC light. Surfaces and gloves were decontaminated using RNase AWAY (Molecular Bio-Products Inc., San Diego, CA).

### 16S rRNA profiling and analysis

To amplify the variable region 4 (V4) of the 16S rRNA gene, we used the 515F and 806R primers (GTGCCAGCMGCCGCGGTAA and GGACTACHVGGGTWTCTAAT respectively) modified to contain Illumina adapters and barcode sequences to allow for directional sequencing (Caporaso et al. 2011, Proc Natl Acad Sci USA, 108 Suppl 1:4516-4522). Sequencing was performed on the Illumina MiSeq platform (MiSeq Reagent Kit v2, 500-cycles, 20% PhiX) according to the manufacturer's specifications to generate paired-end reads of 250 bases in length in each direction. The overlapping paired-end reads were merged using fastq-join (Aronesty et al. 2013, Open Bioinforma J 7:1-8) and processed with LotuS pipeline (Hildebrand et al. 2014, Microbiome 2:30), clustering sequences into OTUs using Greengenes 2013 database for taxonomy assignation. Further analysis was performed in a subset of the data rarefied at 25000 and 20000 observations per sample using phyloseq (McMurdie et al. 2012, Pacific Symposium on Biocomputing 235-246) and R scripts.

### Statistical Analysis

Statistical significance was evaluated using non-parametric Welch Two Sample t-test with 1000 permutations, Mann-Whitney-Wilcoxon Test, Kruskal-Wallis H-test (non-parametric ANOVA) and Spearman rank correlation tests as specified throughout the results. The significance threshold was set at 0.05. Permutational Multivariate analysis of variance (PERMANOVA) was conducted with the vegan functions adonis using 10000 permutations. Correction for multiple testing (q-values) was performed by applying the Benjamini-Hochberg False Discovery Rate (FDR) approach. PERMANOVA and Kruskal-Wallis H-test were performed on filtered OTUs matrices for ileum and colon after removing OTUs with absolute abundance lower than 0.01%.

### Animal housing

TNFΔARE mice were bred and housed in accordance with the general recommendations for animal breeding and housing. All experiments were conducted in conditions according to the guidelines of the Ethics Committee of Laboratory Animals Welfare of Ghent University.

### Clinical scoring of peripheral arthritis

TNFΔARE mice were scored for signs of arthritis of the front and hind paws twice a week as previously described (Jacques et al., Arthritis and Rheumatism, 2010). Briefly, signs of peripheral arthritis were scored as follows: 0 normal, 0.5 1 finger/toe affected, 1swelling of wrist/sagging of footpad, 2 mild swelling and distortion of the metacarpophalangeal/ tarsophalangeal joints, and 3 distortion of all joints. A cumulative score for all paws is calculated between 0 and 12 (4 paws, max score 3).

### Antibiotics treatment

Mice were treated with a broad spectrum antibiotics mix in the drinking water: 200 mg/liter ciprofloxacin, 1 g/liter ampicillin, 1 g/liter metronidazole and 500 mg/liter vancomycin. Antibiotics were refreshed and mice were transferred to clean cages after three days.

### Oral gavages of donor stool samples

Mice were first starved for 4 hours and received 50µl of sterile sodiumbicarbonate 8.4% by oral gavage (20-gauge plastic feeding tube) to neutralize stomach acid. 15 minutes later, a maximum volume of 200µl/25g of body weight of fecal donor solution (1,2 x10e12 CFU bacteria per 200 µl - 20% stool sample in thioglycolate buffer) was administered by oral gavage. One week later, a second gavage was given in a similar procedure.

### TNF ELISA

Blood was collected from the mice through retro-orbital blood collection using a microhematocrit blood tube. The serum was separated and stored at -20 °C until analysis by a classical sandwich ELISA (eBioscience, counting Ab: 14-7423-85, detection Ab: 13-7341-81), as described before (Aspeslagh et al. EMBO 2011). Briefly, purified and biotinylated anti-mouse TNF Abs were obtained from eBioscience and the ELISA assay was performed according to manufacturer's instructions using 0.1M carbonic acid (NaHCO3/Na2CO3; pH9.5) as coating buffer and 0.1% casein as blocking agent. Serum TNF concentrations were calculated by means of standard curves obtained for each cytokine (eBioscience 14-8321-63). In addition, serum TNF levels were confirmed by an alternative approach, using Bio-Plex Pro cytokine set (Bio-Rad 17165023M) according to manufacturer's instructions.

### TABLES

**Table 1. A selection of Dialister OTU's and their corresponding 16S rRNA fragments.**

| **Dialister OTU** | **Highest similarity to** | **16S rRNA fragment** |
|---|---|---|
| #OTU4552 | *D*. *micraerophilus* | |
| #OTU6938 | *D*. *succinatiphilus* | |
| #OTU1378 | Uncultured Dialister | |
| #OTU605 | *Dialister in visus* | |
| #OTU233 | *Dialister invisus* | |
| #OTU62 | *Dialister in visus* | |
| #OTU60 | *Dialister in visus* | |
| #OTU373 | Uncultured Dialister | |
| #OTU_125 1 | *D. invisus* | |
| # OTU_1303 | *D*. *propionifaciens* | |
| # OTU_1459 | Uncultured Dialister | |
| # OTU_2237 | *D. invisus* | |
| # OTU_2284 | *D. invisus* | |
| # OTU_2460 | *D. invisus* | |
| # OTU_2494 | *Dialister invisus* | |
| # OTU_361 | *D*. *succinatiphilus* | |
| # OTU_420 | *D*. *propionifaciens* | |
| # OTU_44 | *D. invisus* | |
| # OTU_497 | *D. invisus* | |
| # OTU_515 | *D*. *propionifaciens* | |
| #OTU_685 | *D*. *succinatiphilus* | |
| #OTU_882 | *D. invisus* | |
| # OTU_982 | *D. pseumosintes* | |
| OTU_984 | *D. pseumosintes* | |

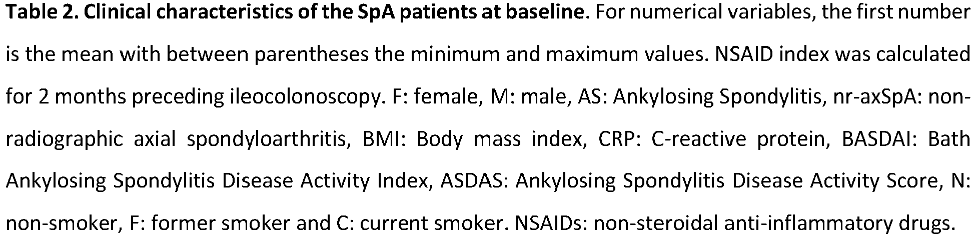

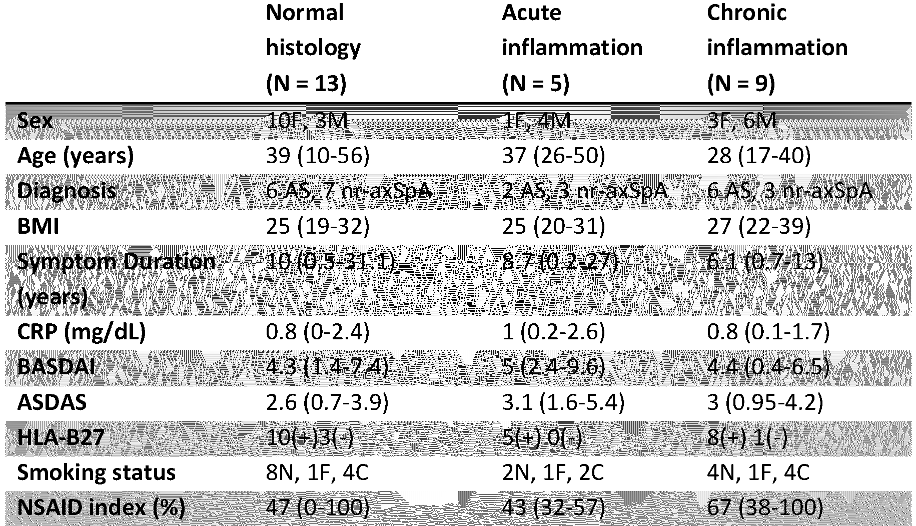

**Table 3. A subselection of Dialister OTU's and their corresponding 16S rRNA fragments selected from Table 1.**

| **Dialister OTU** | **Highest similarity to** | **SEQ ID No.** | **16S rRNA fragment** |
|---|---|---|---|
| #OTU4552 | *D*. *micraerophilus* | 11 | |
| #OTU6938 | *D*. *succinatiphilus* | 12 | |
| #OTU1378 | Uncultured Dialister | 13 | |
| #OTU605 | *Dialister invisus* | 14 | |
| | | | |
| #OTU233 | *Dialister invisus* | 15 | |
| #OTU373 | Uncultured Dialister | 18 | |
| #OTU_1251 | *D. invisus* | 19 | |
| # OTU_1303 | *D*. *propionifaciens* | 20 | |
| # OTU_1459 | Uncultured Dialister | 21 | |
| # OTU_2237 | *D. invisus* | 22 | |
| # OTU_2284 | *D. invisus* | 23 | |
| # OTU_2460 | *D. invisus* | 24 | |
| | | | |
| # OTU_2494 | *Dialister invisus* | 25 | |
| # OTU_361 | *D*. *succinatiphilus* | 26 | |
| # OTU_420 | *D*. *propionifaciens* | 27 | |
| # OTU_497 | *D. invisus* | 29 | |
| # OTU_515 | *D*. *propionifaciens* | 30 | |
| #OTU_685 | *D*. *succinatiphilus* | 31 | |
| #OTU_882 | *D. invisus* | 32 | |
| # OTU_982 | *D. pseumosintes* | 33 | |
| OTU_984 | *D. pseumosintes* | 34 | |

**Table 4. A subselection of Dialister OTU's and their corresponding 16S rRNA fragments selected from Table 1.**

| **Dialister OTU** | **Highest similarity to** | **SEQ ID No.** | **16S rRNA fragment** |
|---|---|---|---|
| #OTU62 | *Dialister invisus* | 16 | |
| #OTU60 | *Dialister invisus* | 17 | |
| # OTU_44 | *D. invisus* | 28 | |

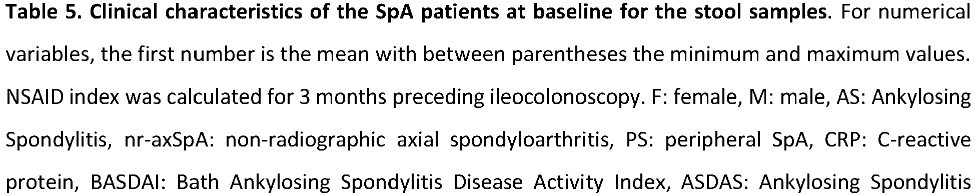

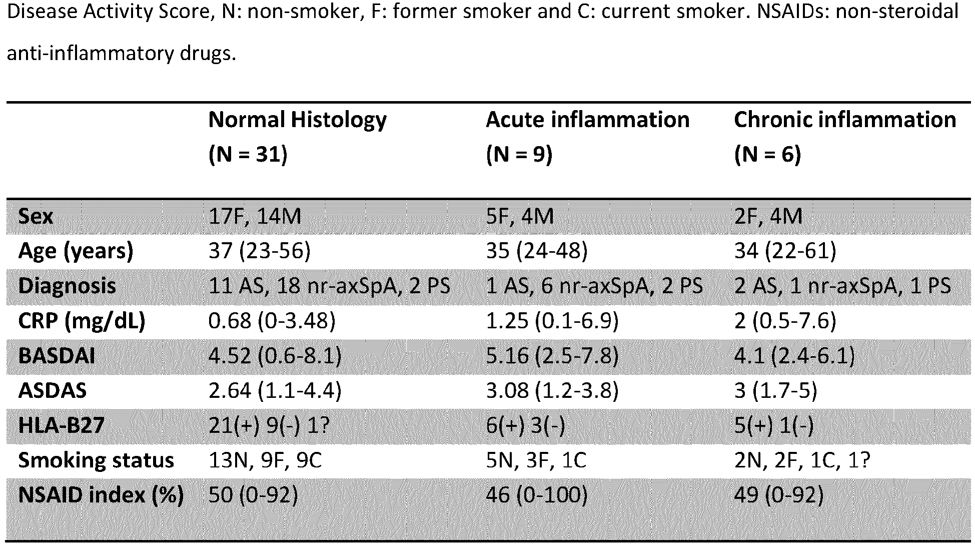

## Claims

1. A composition comprising at least one Dialister spp. for use in treating, preventing or reducing the severity of spondyloarthritis (SpA) or of gut and/or joint inflammation in SpA patients, wherein the at least one Dialister spp. is selected from the list consisting of OTU4552 comprising SEQ ID No. 11, OTU6938 comprising SEQ ID No. 12, OTU1378 comprising SEQ ID No. 13, OTU605 comprising SEQ ID No. 14 and OTU223 comprising SEQ ID No. 15.

2. The composition according to claim 1 for use according to claim 1, wherein said at least one Dialister spp. originates from bacterial collections.

3. The composition according to claim 2 for use according to claim 1, wherein said bacterial collections are obtained from fecal samples and/or microbiota obtained from intestinal biopsies or other clinical biopsies.

4. The composition according to claim 3 for use according to claim 1, wherein said fecal samples and/or biopsies are obtained from a healthy subject.

5. The composition according to any of claims 1-4 for use according to claim 1, wherein said composition is a fecal transplant or an oral formulation.

6. The composition of any of the preceding claims for use according to claim 1, wherein the composition further comprises an anti-inflammatory and/or an analgesic drug.

7. The composition of any of the preceding claims for use according to claim 1, wherein SpA is selected from ankylosing spondylitis, non-radiographic axial SpA, reactive arthritis, psoriatic arthritis, enteropathic arthritis, undifferentiated spondyloarthritis, juvenile idiopathic arthritis or juvenile-onset SpA.

8. A screening method to identify a compound that reduces the intestinal abundance of disease associated *Dialister* spp. selected from OTU60 comprising SEQ ID No. 57 or OTU62 comprising SEQ ID No. 56, said method comprising the following steps:
- contacting isolated disease associated *Dialister* spp. from subjects suffering from or at risk of SpA or of gut and/or joint inflammation with a compound in an *in vitro* environment;
- selecting a compound that inhibits growth of at least one of said disease associated *Dialister* spp.

9. The method of claim 8 wherein SpA is selected from ankylosing spondylitis, non-radiographic axial SpA, reactive arthritis, psoriatic arthritis, enteropathic arthritis, undifferentiated spondyloarthritis, juvenile idiopathic arthritis or juvenile-onset SpA.

## Patentansprüche

1. Zusammensetzung, umfassend mindestens eine Dialister spp., zur Verwendung bei der Behandlung, Prävention oder Verringerung der Schwere von Spondyloarthritis (SpA) oder einer Darm- und/oder Gelenkentzündung bei SpA-Patienten, wobei die mindestens eine Dialister spp. aus der Liste bestehend aus OTU4552, umfassend SEQ ID Nr. 11, OTU6938, umfassend SEQ ID Nr. 12, OTU1378, umfassend SEQ ID Nr. 13, OTU605, umfassend SEQ ID Nr. 14, und OTU223, umfassend SEQ ID Nr. 15, ausgewählt ist.

2. Zusammensetzung nach Anspruch 1 zur Verwendung nach Anspruch 1, wobei die mindestens eine Dialister spp. von Bakterienentnahmen stammt.

3. Zusammensetzung nach Anspruch 2 zur Verwendung nach Anspruch 1, wobei die Bakterienentnahmen aus Stuhlproben und/oder Mikrobiota, die aus Darmbiopsien oder anderen klinischen Biopsien erhalten werden, erhalten werden.

4. Zusammensetzung nach Anspruch 3 zur Verwendung nach Anspruch 1, wobei die Stuhlproben und/oder die Biopsien von einem gesunden Probanden erhalten werden.

5. Zusammensetzung nach einem der Ansprüche 1-4 zur Verwendung nach Anspruch 1, wobei die Zusammensetzung eine Stuhltransplantation oder eine orale Formulierung ist.

6. Zusammensetzung nach einem der vorhergehenden Ansprüche zur Verwendung nach Anspruch 1, wobei die Zusammensetzung weiterhin einen entzündungshemmenden und/oder einen schmerzlindernden Wirkstoff umfasst.

7. Zusammensetzung nach einem der vorhergehenden Ansprüche zur Verwendung nach Anspruch 1, wobei die SpA aus Morbus Bechterew, nicht-röntgenologischer axialer SpA, reaktiver Arthritis, psoriatischer Arthritis, enteropathischer Arthritis, undifferenzierter Spondyloarthritis, juveniler idiopathischer Arthritis oder SpA im Jugendalter ausgewählt ist.

8. Screening-Verfahren zur Identifikation einer Verbindung, die die Darm-Abundanz von krankheitsassoziierter *Dialister* spp., ausgewählt aus OTU60, umfassend SEQ ID Nr. 57, oder OTU62, umfassend SEQ ID Nr. 56, verringert, wobei das Verfahren die folgenden Schritte umfasst:
- Inkontaktbringen von isolierter krankheitsassoziierter *Dialister* spp. von Probanden, die an SpA oder einer Darm- und/oder Gelenkentzündung leiden oder ein Risiko für diese aufweisen, mit einer Verbindung in einer In-vitro-Umgebung;
- Auswählen einer Verbindung, die das Wachstum von mindestens einer der krankheitsassoziierten *Dialister* spp. hemmt.

9. Verfahren nach Anspruch 8, wobei die SpA aus Morbus Bechterew, nicht-röntgenologischer axialer SpA, reaktiver Arthritis, psoriatischer Arthritis, enteropathischer Arthritis, undifferenzierter Spondyloarthritis, juveniler idiopathischer Arthritis oder SpA im Jugendalter ausgewählt ist.

## Revendications

1. Composition comprenant au moins une Dialister spp. destinée à être utilisée dans le traitement, la prévention ou la réduction de la gravité d'une spondylarthrite (SpA) ou d'une inflammation intestinale et/ou articulaire chez des patients atteints de SpA, dans laquelle l'au moins une Dialister spp. est sélectionnée parmi la liste constituée de OTU4552 comprenant SEQ ID N° 11, OTU6938 comprenant SEQ ID N° 12, OTU1378 comprenant SEQ ID N° 13, OTU605 comprenant SEQ ID N° 14 et OTU223 comprenant SEQ ID N° 15.

2. Composition selon la revendication 1 destinée à être utilisée selon la revendication 1, dans laquelle ladite au moins une Dialister spp. provient de collections bactériennes.

3. Composition selon la revendication 2 destinée à être utilisée selon la revendication 1, dans laquelle lesdites collections bactériennes sont obtenues à partir d'échantillons fécaux et/ou de microbiote obtenu à partir de biopsies intestinales ou d'autres biopsies cliniques.

4. Composition selon la revendication 3 destinée à être utilisée selon la revendication 1, dans laquelle lesdit(e)s échantillons fécaux et/ou biopsies sont obtenu(e)s auprès d'un sujet sain.

5. Composition selon l'une quelconque des revendications 1 à 4 destinée à être utilisée selon la revendication 1, dans laquelle ladite composition est une transplantation fécale ou une formulation orale.

6. Composition selon l'une quelconque des revendications précédentes destinée à être utilisée selon la revendication 1, dans laquelle la composition comprend en outre un anti-inflammatoire et/ou un analgésique.

7. Composition selon l'une quelconque des revendications précédentes destinée à être utilisée selon la revendication 1, dans laquelle la SpA est sélectionnée parmi la spondylarthrite ankylosante, la SpA axiale non radiographique, l'arthrite réactionnelle, l'arthrite psoriasique, l'arthrite entéropathique, la spondylarthrite indifférenciée, l'arthrite juvénile idiopathique ou la SpA juvénile.

8. Procédé de criblage pour identifier un composé qui réduit l'abondance intestinale de *Dialister* spp. associée à la maladie sélectionnée parmi OTU60 comprenant SEQ ID N° 57 ou OTU62 comprenant SEQ ID N° 56, ledit procédé comprenant les étapes suivantes :
- mettre en contact de la *Dialister* spp. associée à la maladie provenant de sujets souffrant de ou à risque de SpA ou d'inflammation intestinale et/ou articulaire avec un composé dans un environnement *in vitro* ;
- sélectionner un composé qui inhibe la croissance d'au moins une desdites *Dialister* spp. associées à la maladie.

9. Procédé selon la revendication 8, dans lequel la SpA est sélectionnée parmi la spondylarthrite ankylosante, la SpA axiale non radiographique, l'arthrite réactionnelle, l'arthrite psoriasique, l'arthrite entéropathique, la spondylarthrite indifférenciée, l'arthrite juvénile idiopathique ou la SpA juvénile.


### SEQUENCE LISTING

<110> VIB VZW UNIVERSITEIT GENT KATHOLIEKE UNIVERSITEIT LEUVEN, K.U.LEUVEN R&D
<120> MEANS AND METHODS TO TREAT INFLAMMATION-ASSOCIATED DISORDERS OR CONDITIONS
<130> JD/DIATH/556
<150> EP 16176039.2
   <151> 2016-06-23
<160> 58
<170> PatentIn version 3.5
<210> 1
   <211> 21
   <212> DNA
   <213> Artificial Sequence
<220>
   <223> Primer
<400> 1
   ccatgaagtc ggaatcgcta g 21
<210> 2
   <211> 16
   <212> DNA
   <213> Artificial Sequence
<220>
   <223> Primer
<400> 2
   gcttgacggg cggtgt 16
<210> 3
   <211> 20
   <212> DNA
   <213> Artificial Sequence
<220>
   <223> Primer
<400> 3
   gaggggtttg cgactgatta 20
<210> 4
   <211> 20
   <212> DNA
   <213> Artificial Sequence
<220>
   <223> Primer
<400> 4
   ccgtcagact ttcgtccatt 20
<210> 5
   <211> 20
   <212> DNA
   <213> Artificial Sequence
<220>
   <223> Primer
<400> 5
   gagcctctaa ccagttccag 20
<210> 6
   <211> 20
   <212> DNA
   <213> Artificial Sequence
<220>
   <223> Primer
<400> 6
   tgagtgtggc tatgacttcg 20
<210> 7
   <211> 20
   <212> DNA
   <213> Artificial Sequence
<220>
   <223> Primer
<400> 7
   cgggcaggct catcggtgtt 20
<210> 8
   <211> 20
   <212> DNA
   <213> Artificial Sequence
<220>
   <223> Primer
<400> 8
   ttcgcccgta gcccccagta 20
<210> 9
   <211> 19
   <212> DNA
   <213> Artificial Sequence
<220>
   <223> Primer
<400> 9
   gtgccagcmg ccgcggtaa 19
<210> 10
   <211> 20
   <212> DNA
   <213> Artificial Sequence
<220>
   <223> Primer
<400> 10
   ggactachvg ggtwtctaat 20
<210> 11
   <211> 252
   <212> DNA
   <213> Dialister micraerophilus
<400> 11
   tacgtaggtg gcaagcgttg tccggaatta ttgggcgtaa agcgcgcgca ggcggcttct 60
   taagtccatc ttaaaagtgc ggggcttaac cccgtgatgg gatggaaact gggaggctgg 120
   agtatcggag aggaaagtgg aattcctagt gtagcggtga aatgcgtaga gattaggaag 180
   aacaccggtg gcgaaggcga ctttctggac gacaactgac gctgaggcgc gaaagcgtgg 240
   ggagcaaaca gg 252
<210> 12
   <211> 252
   <212> DNA
   <213> Dialister succinatiphilus
<400> 12
   tacgtaggtg gcaagcgttg tccggaatta ttgggcgtaa agcgcgcgca ggcggctttc 60
   taagtccatc ttaaaagtgc ggggcttaac cccgtgatgg gatggaaact ggaaagctgg 120
   agtatcggag aggaaagtgg aattcctagt gtagcggtga aatgcgtaga gattaggaag 180
   aacaccggtg gcgaaggcga ctttctggac gacaactgac gttgaggcac gaaagcgtgg 240
   gtatcgaaca gg 252
<210> 13
   <211> 252
   <212> DNA
   <213> Dialister sp.
<400> 13
   tacgtatggt gcaagcgttg tccggaatta ttgggcgtaa agcgcgcgca ggcggctttc 60
   taagtccatc ttaaaagtgc ggggcttaac cccgtgatgg gaaggaaact gggaagctgg 120
   agtatcggag aggaaagtgg aattcctagt gtagcggtga aatgcgtaga gattaggaag 180
   aacaccggtg gcgaaggcga ctttctggac gaaaactgac gctgaggcgc gaaagcgtgg 240
   ggagcaaaca gg 252
<210> 14
   <211> 252
   <212> DNA
   <213> Dialister invisus
<400> 14
   aacgtaggtc acaagcgttg tccggaatta ttgggcgtaa agcgcgcgca ggcggcttcc      60
   caagtccctc ttaaaagtgc ggggcttaac cccgtgatgg gaaggaaact gggaagctgg     120
   agtatcggag aggaaagtgg aattcctagt gtagcggtga aatgcgtaga gattaggaag     180
   aacaccggtg gcgaaggcga ctttctggac gaaaactgac gctgaggcgc gaaagcgtgg     240
   ggagcaaaca gg                                                         252
<210> 15
   <211> 252
   <212> DNA
   <213> Dialister invisus
<400> 15
   tacgtaggtg gcaagcgttg tccggaatta ttgggcgtaa agcgcgcgca ggcggcttcc      60
   caagtccctc ttaaaagtgc ggggcttaac cccgtgatgg gaaggaaact gggaagctgg     120
   agtatcggag aggaaagtgg aattcctagt gtagcggtga aatgcgtaga gattaggaag     180
   aacaccggtg gcgaaggcga ctttctggac gaaaactgac gctgaggcgc gaaagtgcgg     240
   gtatcgaaca gg                                                         252
<210> 16
   <211> 272
   <212> DNA
   <213> Dialister invisus
<400> 16
   agtgtcagcc gccgcggtaa tacgtaggtg gcaagcgttg tccggaatta ttgggcgtaa      60
   agcgcgcgca ggcggcttcc caagtccctc ttaaaagtgc ggggcttaac cccgtgatgg     120
   gaaggaaact gggaagctgg agtatcggag aggaaagtgg aattcctagt gtagcggtga     180
   aatgcgtaga gattaggaag aacaccggtg gcgaaggcga ctttctggac gaaaactgac     240
   gctgaggcgc gaaagcgtgg ggagcaaaca gg                                   272
<210> 17
   <211> 249
   <212> DNA
   <213> Dialister invisus
<400> 17
   acgtaggtgg caagcgttgt ccggaattat tgggcgtaaa gcgcgcgcag gcggcttccc 60
   aagtccctct taaaagtgcg gggcttaacc ccgtgaggga aggaaactgg gaagctggag 120
   tatcggagag gaaagtggaa ttcctagtgt agcggtgaaa tgcgtagaga ttaggaagaa 180
   caccggtggc gaaggcgact ttctggacga aaactgacgc tgaggcgcga aagcgtgggg 240
   agcaaacag 249
<210> 18
   <211> 250
   <212> DNA
   <213> Dialister sp.
<400> 18
   acgtaggtgg caagcgttgt ccggaattat tgggcgtaaa gcgcgcgcag gcggcttccc 60
   aagtccctct taaaagtgcg gggcttaacc ccgtgatggg aaggaaactg ggaagctgga 120
   gtatcggaga ggaaagtgga attcctagtg tagcggtgaa atgcgtagat attaggagga 180
   acaccagtgg cgaaggcggc ttactggaca gtaactgacg ttgaggctcg aaagcgtggg 240
   gagcaaacag 250
<210> 19
   <211> 252
   <212> DNA
   <213> Dialister invisus
<400> 19
   cctgtttgct ccccacgctt tcgtgcctca gtgtcagtag cagtccagta agtcgccttc 60
   gccactggtg ttcctcctaa tatctacgca tttcaccgct acactaggaa ttccacttac 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 20
   <211> 252
   <212> DNA
   <213> Dialister propionifaciens
<400> 20
   cctgtttgct ccccacgctt tcgtgcctca gtgtcagtag cagtccagta agtcgccttc      60
   gccactggtg ttcttcctaa tctctacgca tttcaccgct acactaggaa ttccactttc     120
   ctctccgata ctccagcctc tcagtttcca tcccatcacg gggttaagcc ccgcactttt     180
   aagatggact taagaagccg cctgcgcgcg ctttacgccc aataattccg gacaacgctt     240
   gccacctacg ta                                                         252
<210> 21
   <211> 252
   <212> DNA
   <213> Dialister sp.
<400> 21
   cctgtttgct ccccacgctt tcgcgcctca gcgtcagttt tcgtccagaa agtcgccttc      60
   gccaccggtg ttcttcctaa tatctacgca tttcaccgct acactaggaa ttccaccttc     120
   ctctccgata ctccagtctc ccagtttcca tcccctcacg gggttaagcc ccgcacttct     180
   aagatagact taagagaccg cctgcgcgcg ctttacgccc aataattccg gacaacgctt     240
   gccacctacg ta                                                         252
<210> 22
   <211> 252
   <212> DNA
   <213> Dialister invisus
<400> 22
   cctgttcgct cccctagctt tcgcgcctca gcgtcagtgg cggcccagaa ggctgccttc      60
   gccatcggtg ttcttcccaa tatctgcgca tttcaccgct acactgggaa ttccactttc     120
   ctctccgata ctccagcttc ccagtttcct tcccatcacg gggttaagcc ccgcactttt     180
   aagagggact tgggaagccg cctgcgcgcg ctttacgccc aataattccg gacaacgctt     240
   gccacctacg ta                                                         252
<210> 23
   <211> 252
   <212> DNA
   <213> Dialister invisus
<400> 23
   cctgtttgat acccacactt tcgagcctca atgtcagttg cagcttagca ggctgccttc 60
   gcaatcggag ttcttcgtga tatctaagca tttcaccgct acactaggaa ttccactttc 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 24
   <211> 252
   <212> DNA
   <213> Dialister invisus
<400> 24
   cctgtttgct ccccacactt tcgcgcctca gcgtcagtta cagtccagtt agtcgccttc 60
   gccactggtg ttcctcccaa tctctacgca tttcaccgct acactgggaa ttccactaac 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 25
   <211> 252
   <212> DNA
   <213> Dialister invisus
<400> 25
   cctgttcgct ccccacgctt tcgagcctca gcgtcagtta cagaccagag agccgctttc 60
   gccaccggtg ttcctccata tatctacgca tttcaccgct acacatggaa ttccactttc 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 26
   <211> 252
   <212> DNA
   <213> Dialister succinatiphilus
<400> 26
   cctgtttgct ccccacgctt tcgcgcctca gcgtcagttg tcgtccagaa agccgccttc 60
   gccaccggtg ttcttcctaa tctctacgca tttcaccgct acactaggaa ttccgctttc 120
   ctctccgata ctccagctct tcagtttcca tcccatcatg gggttaagcc ccacgctttt 180
   aagatggact taaagggccg cctgcgcgcg ctttacgccc aataattccg gacaacgctt 240
   gccacctacg ta 252
<210> 27
   <211> 252
   <212> DNA
   <213> Dialister propionifaciens
<400> 27
   cctgtttgct ccccacgctt tcgagcctca gcgtcagtta tcgtccagta agccgccttc 60
   gccactggtg ttcctcctaa tatctacgca tttcaccgct acactaggaa ttccactttc 120
   ctctccgata ctccagcctc tcagtttcca tcccatcacg gggttaagcc ccgcactttt 180
   aagatggact taagaagccg cctgcgcgcg ctttacgccc aataattccg gacaacgctt 240
   gccacctacg ta 252
<210> 28
   <211> 252
   <212> DNA
   <213> Dialister invisus
<400> 28
   cctgtttgct ccccacgctt tcgcgcctca gcgtcagttt tcgtccagaa agtcgccttc 60
   gccaccggtg ttcttcctaa tctctacgca tttcaccgct acactaggaa ttccactttc 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 29
   <211> 252
   <212> DNA
   <213> Dialister invisus
<400> 29
   cctgtttgct ccccacgctt tcgagcctca acgtcagtta ccgtccagta agccgccttc 60
   gccactggtg ttcctcctaa tatctacgca tttcaccgct acactaggaa ttccgcttac 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 30
   <211> 252
   <212> DNA
   <213> Dialister propionifaciens
<400> 30
   cctgtttgct acccacactt tcgtgcctca gcgtcagttg tcgtccagaa agtcgccttc 60
   gccaccggtg ttcttcctaa tctctacgca tttcaccgct acactaggaa ttccactttc 120
   ctctccgata ctccagcctc ccagtttcca tcccatcacg gggttaagcc ccgcactttt 180
   aagatggact taagaagccg cctgcgcgcg ctttacgccc aataattccg gacaacgctt 240
   gccacctacg ta 252
<210> 31
   <211> 252
   <212> DNA
   <213> Dialister succinatiphilus
<400> 31
   cctgtttgct ccccacgctt tcgagcctca acgtcagtca tcgtccagca agccgccttc 60
   gccactggtg ttcctcctaa tatctacgca tttcaccgct acactaggaa ttccactttc 120
   ctctccgata ctccagcttt ccagtttcca tcccatcacg gggttaagcc ccgcactttt 180
   aagatggact tagaaagccg cctgcgcgcg ctttacgccc aataattccg gacaacgctt 240
   gccacctacg ta 252
<210> 32
   <211> 252
   <212> DNA
   <213> Dialister invisus
<400> 32
   cctgtttgct ccccacgctt tcgagcctca gcgtcagtta aagcccagta agccgccttc 60
   gccactgatg ttcctcctaa tatctacgca tttcaccgct acactaggaa ttccactttc 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 33
   <211> 252
   <212> DNA
   <213> Dialister pseumosintes
<400> 33
   cccgttcgct cccctggctt tcgcgcctca gcgtcagttt tcgtccagaa agtcgccttc 60
   gccactggtg ttcttcctaa tatctacgca tttcaccgct acactaggaa ttccactttc 120
   ctctccgata ctccagcttc ccagtttcca tcccctcatg gggttgagcc ccacgctttt 180
   aagatggact taagaaaccg cctgcgcgcg ctttacgccc aataattccg gacaacgctt 240
   gccacctacg ta 252
<210> 34
   <211> 252
   <212> DNA
   <213> Dialister pseumosintes
<400> 34
   cctgtttgct ccccacgctt tcgcgcctca gcgtcagttt tcgtctagaa agtcgccttc 60
   gccaccggtg ttcttcctaa tctctacgca tttcaccgct acactaggaa ttccactttc 120
   ccctccgata ctccagcttc ccagtttcca tcccctcatg gggttgagcc ccacgctttt 180
   aagatggact taagaaaccg cctgcgcgcg ctttacgccc aataattccg gacaacgctt 240
   gccacctacg ta 252
<210> 35
   <211> 252
   <212> DNA
   <213> Dialister micraerophilus
<400> 35
   tacgtaggtg gcaagcgttg tccggaatta ttgggcgtaa agcgcgcgca ggcggcttct 60
   taagtccatc ttaaaagtgc ggggcttaac cccgtgatgg gatggaaact gggaggctgg 120
   agtatcggag aggaaagtgg aattcctagt gtagcggtga aatgcgtaga gattaggaag 180
   aacaccggtg gcgaaggcga ctttctggac gacaactgac gctgaggcgc gaaagcgtgg 240
   ggagcaaaca gg 252
<210> 36
   <211> 252
   <212> DNA
   <213> Dialister succinatiphilus
<400> 36
   tacgtaggtg gcaagcgttg tccggaatta ttgggcgtaa agcgcgcgca ggcggctttc      60
   taagtccatc ttaaaagtgc ggggcttaac cccgtgatgg gatggaaact ggaaagctgg     120
   agtatcggag aggaaagtgg aattcctagt gtagcggtga aatgcgtaga gattaggaag     180
   aacaccggtg gcgaaggcga ctttctggac gacaactgac gttgaggcac gaaagcgtgg     240
   gtatcgaaca gg                                                         252
<210> 37
   <211> 252
   <212> DNA
   <213> Dialister sp.
<400> 37
   tacgtatggt gcaagcgttg tccggaatta ttgggcgtaa agcgcgcgca ggcggctttc      60
   taagtccatc ttaaaagtgc ggggcttaac cccgtgatgg gaaggaaact gggaagctgg     120
   agtatcggag aggaaagtgg aattcctagt gtagcggtga aatgcgtaga gattaggaag     180
   aacaccggtg gcgaaggcga ctttctggac gaaaactgac gctgaggcgc gaaagcgtgg     240
   ggagcaaaca gg                                                         252
<210> 38
   <211> 252
   <212> DNA
   <213> Dialister invisus
<400> 38
   aacgtaggtc acaagcgttg tccggaatta ttgggcgtaa agcgcgcgca ggcggcttcc      60
   caagtccctc ttaaaagtgc ggggcttaac cccgtgatgg gaaggaaact gggaagctgg     120
   agtatcggag aggaaagtgg aattcctagt gtagcggtga aatgcgtaga gattaggaag     180
   aacaccggtg gcgaaggcga ctttctggac gaaaactgac gctgaggcgc gaaagcgtgg     240
   ggagcaaaca gg                                                         252
<210> 39
   <211> 252
   <212> DNA
   <213> Dialister invisus
<400> 39
   tacgtaggtg gcaagcgttg tccggaatta ttgggcgtaa agcgcgcgca ggcggcttcc 60
   caagtccctc ttaaaagtgc ggggcttaac cccgtgatgg gaaggaaact gggaagctgg 120
   agtatcggag aggaaagtgg aattcctagt gtagcggtga aatgcgtaga gattaggaag 180
   aacaccggtg gcgaaggcga ctttctggac gaaaactgac gctgaggcgc gaaagtgcgg 240
   gtatcgaaca gg 252
<210> 40
   <211> 250
   <212> DNA
   <213> Dialister sp.
<400> 40
   acgtaggtgg caagcgttgt ccggaattat tgggcgtaaa gcgcgcgcag gcggcttccc 60
   aagtccctct taaaagtgcg gggcttaacc ccgtgatggg aaggaaactg ggaagctgga 120
   gtatcggaga ggaaagtgga attcctagtg tagcggtgaa atgcgtagat attaggagga 180
   acaccagtgg cgaaggcggc ttactggaca gtaactgacg ttgaggctcg aaagcgtggg 240
   gagcaaacag 250
<210> 41
   <211> 252
   <212> DNA
   <213> Dialister invisus
<400> 41
   cctgtttgct ccccacgctt tcgtgcctca gtgtcagtag cagtccagta agtcgccttc 60
   gccactggtg ttcctcctaa tatctacgca tttcaccgct acactaggaa ttccacttac 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 42
   <211> 252
   <212> DNA
   <213> Dialister propionifaciens
<400> 42
   cctgtttgct ccccacgctt tcgtgcctca gtgtcagtag cagtccagta agtcgccttc 60
   gccactggtg ttcttcctaa tctctacgca tttcaccgct acactaggaa ttccactttc 120
   ctctccgata ctccagcctc tcagtttcca tcccatcacg gggttaagcc ccgcactttt 180
   aagatggact taagaagccg cctgcgcgcg ctttacgccc aataattccg gacaacgctt 240
   gccacctacg ta 252
<210> 43
   <211> 252
   <212> DNA
   <213> Dialister sp.
<400> 43
   cctgtttgct ccccacgctt tcgcgcctca gcgtcagttt tcgtccagaa agtcgccttc 60
   gccaccggtg ttcttcctaa tatctacgca tttcaccgct acactaggaa ttccaccttc 120
   ctctccgata ctccagtctc ccagtttcca tcccctcacg gggttaagcc ccgcacttct 180
   aagatagact taagagaccg cctgcgcgcg ctttacgccc aataattccg gacaacgctt 240
   gccacctacg ta 252
<210> 44
   <211> 252
   <212> DNA
   <213> Dialister invisus
<400> 44
   cctgttcgct cccctagctt tcgcgcctca gcgtcagtgg cggcccagaa ggctgccttc 60
   gccatcggtg ttcttcccaa tatctgcgca tttcaccgct acactgggaa ttccactttc 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 45
   <211> 252
   <212> DNA
   <213> Dialister invisus
<400> 45
   cctgtttgat acccacactt tcgagcctca atgtcagttg cagcttagca ggctgccttc 60
   gcaatcggag ttcttcgtga tatctaagca tttcaccgct acactaggaa ttccactttc 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 46
   <211> 252
   <212> DNA
   <213> Dialister invisus
<400> 46
   cctgtttgct ccccacactt tcgcgcctca gcgtcagtta cagtccagtt agtcgccttc 60
   gccactggtg ttcctcccaa tctctacgca tttcaccgct acactgggaa ttccactaac 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 47
   <211> 252
   <212> DNA
   <213> Dialister invisus
<400> 47
   cctgttcgct ccccacgctt tcgagcctca gcgtcagtta cagaccagag agccgctttc 60
   gccaccggtg ttcctccata tatctacgca tttcaccgct acacatggaa ttccactttc 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 48
   <211> 252
   <212> DNA
   <213> Dialister succinatiphilus
<400> 48
   cctgtttgct ccccacgctt tcgcgcctca gcgtcagttg tcgtccagaa agccgccttc 60
   gccaccggtg ttcttcctaa tctctacgca tttcaccgct acactaggaa ttccgctttc 120
   ctctccgata ctccagctct tcagtttcca tcccatcatg gggttaagcc ccacgctttt 180
   aagatggact taaagggccg cctgcgcgcg ctttacgccc aataattccg gacaacgctt 240
   gccacctacg ta 252
<210> 49
   <211> 252
   <212> DNA
   <213> Dialister propionifaciens
<400> 49
   cctgtttgct ccccacgctt tcgagcctca gcgtcagtta tcgtccagta agccgccttc 60
   gccactggtg ttcctcctaa tatctacgca tttcaccgct acactaggaa ttccactttc 120
   ctctccgata ctccagcctc tcagtttcca tcccatcacg gggttaagcc ccgcactttt 180
   aagatggact taagaagccg cctgcgcgcg ctttacgccc aataattccg gacaacgctt 240
   gccacctacg ta 252
<210> 50
   <211> 252
   <212> DNA
   <213> Dialister invisus
<400> 50
   cctgtttgct ccccacgctt tcgagcctca acgtcagtta ccgtccagta agccgccttc 60
   gccactggtg ttcctcctaa tatctacgca tttcaccgct acactaggaa ttccgcttac 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 51
   <211> 252
   <212> DNA
   <213> Dialister propionifaciens
<400> 51
   cctgtttgct acccacactt tcgtgcctca gcgtcagttg tcgtccagaa agtcgccttc 60
   gccaccggtg ttcttcctaa tctctacgca tttcaccgct acactaggaa ttccactttc 120
   ctctccgata ctccagcctc ccagtttcca tcccatcacg gggttaagcc ccgcactttt 180
   aagatggact taagaagccg cctgcgcgcg ctttacgccc aataattccg gacaacgctt 240
   gccacctacg ta 252
<210> 52
   <211> 252
   <212> DNA
   <213> Dialister succinatiphilus
<400> 52
   cctgtttgct ccccacgctt tcgagcctca acgtcagtca tcgtccagca agccgccttc 60
   gccactggtg ttcctcctaa tatctacgca tttcaccgct acactaggaa ttccactttc 120
   ctctccgata ctccagcttt ccagtttcca tcccatcacg gggttaagcc ccgcactttt 180
   aagatggact tagaaagccg cctgcgcgcg ctttacgccc aataattccg gacaacgctt 240
   gccacctacg ta 252
<210> 53
   <211> 252
   <212> DNA
   <213> Dialister invisus
<400> 53
   cctgtttgct ccccacgctt tcgagcctca gcgtcagtta aagcccagta agccgccttc 60
   gccactgatg ttcctcctaa tatctacgca tttcaccgct acactaggaa ttccactttc 120
   ctctccgata ctccagcttc ccagtttcct tcccatcacg gggttaagcc ccgcactttt 180
   aagagggact tgggaagccg cctgcgcgcg ctttacgccc aataattccg gacaacgctt 240
   gccacctacg ta 252
<210> 54
   <211> 252
   <212> DNA
   <213> Dialister pseumosintes
<400> 54
   cccgttcgct cccctggctt tcgcgcctca gcgtcagttt tcgtccagaa agtcgccttc 60
   gccactggtg ttcttcctaa tatctacgca tttcaccgct acactaggaa ttccactttc 120
   ctctccgata ctccagcttc ccagtttcca tcccctcatg gggttgagcc ccacgctttt 180
   aagatggact taagaaaccg cctgcgcgcg ctttacgccc aataattccg gacaacgctt 240
   gccacctacg ta 252
<210> 55
   <211> 252
   <212> DNA
   <213> Dialister pseumosintes
<400> 55
   cctgtttgct ccccacgctt tcgcgcctca gcgtcagttt tcgtctagaa agtcgccttc 60
   gccaccggtg ttcttcctaa tctctacgca tttcaccgct acactaggaa ttccactttc 120
   ccctccgata ctccagcttc ccagtttcca tcccctcatg gggttgagcc ccacgctttt 180
   aagatggact taagaaaccg cctgcgcgcg ctttacgccc aataattccg gacaacgctt 240
   gccacctacg ta 252
<210> 56
   <211> 272
   <212> DNA
   <213> Dialister invisus
<400> 56
   agtgtcagcc gccgcggtaa tacgtaggtg gcaagcgttg tccggaatta ttgggcgtaa 60
   agcgcgcgca ggcggcttcc caagtccctc ttaaaagtgc ggggcttaac cccgtgatgg 120
   gaaggaaact gggaagctgg agtatcggag aggaaagtgg aattcctagt gtagcggtga 180
   aatgcgtaga gattaggaag aacaccggtg gcgaaggcga ctttctggac gaaaactgac 240
   gctgaggcgc gaaagcgtgg ggagcaaaca gg 272
<210> 57
   <211> 249
   <212> DNA
   <213> Dialister invisus
<400> 57
   acgtaggtgg caagcgttgt ccggaattat tgggcgtaaa gcgcgcgcag gcggcttccc 60
   aagtccctct taaaagtgcg gggcttaacc ccgtgaggga aggaaactgg gaagctggag 120
   tatcggagag gaaagtggaa ttcctagtgt agcggtgaaa tgcgtagaga ttaggaagaa 180
   caccggtggc gaaggcgact ttctggacga aaactgacgc tgaggcgcga aagcgtgggg 240
   agcaaacag 249
<210> 58
   <211> 252
   <212> DNA
   <213> Dialister invisus
<400> 58
   cctgtttgct ccccacgctt tcgcgcctca gcgtcagttt tcgtccagaa agtcgccttc     60
   gccaccggtg ttcttcctaa tctctacgca tttcaccgct acactaggaa ttccactttc    120
   ctctccgata ctccagcttc ccagtttcct tcccatcacg gggttaagcc ccgcactttt    180
   aagagggact tgggaagccg cctgcgcgcg ctttacgccc aataattccg gacaacgctt    240
   gccacctacg ta                                                         252